# EUROPÄISCHE PATENTSCHRIFT

(11) **EP 3 210 637 B1**
(45) Veröffentlichungstag und Bekanntmachung des Hinweises auf die Patenterteilung: **27.01.2021**
(21) Anmeldenummer: 16198978.5
(22) Anmeldetag: 03.04.2002
(51) Int. Cl.: A61M 5/158, A61M 25/06, A61M 39/02

(54) **INFUSIONSSET**
INFUSION SET
DISPOSITIF DE PERFUSION

(30) Priorität: 06.04.2001 DE 10117285; 19.06.2001 DE 20110059 U
(43) Veröffentlichungstag der Anmeldung: 30.08.2017
(62) Teilanmeldung aus: 02704544.2
(73) Patentinhaber: F. Hoffmann-La Roche AG, 4070 Basel (CH); Roche Diabetes Care GmbH, 68305 Mannheim (DE)
(72) Erfinder: HUNN, Marcel, 3400 Burgdorf (CH); LINIGER, Jürg, 3072 Ostermundigen (CH); DENOTH, Patrik, 1797 Münchenwiler (CH); OESCH, Marc, 3326 Krauchthal (CH); BÜTIKOFER, Markus, 3665 Wattenwil (CH); ZIHLMANN, Rudolf, 3550 Langnau (CH); SCHEURER, Simon, 3006 Bern (CH)
(74) Vertreter: Rentsch Partner AG

(56) Entgegenhaltungen:
- EP-A- 0 258 580
- EP-A- 0 956 879
- EP-A2- 0 615 768
- WO-A-99/33504
- WO-A2-02/053220
- WO-A2-02/094352
- WO-A2-03/075980
- DE-A1- 19 912 459
- DE-U1- 29 905 069
- US-A- 5 960 797

## Beschreibung

Die vorliegende Erfindung bezieht sich auf System zum Verbinden einer Flüssigkeitszufuhr mit einer Kanüle.

Aus der EP 0 451 040 A1 ist eine Vorrichtung zum Einbringen eines Katheters mit einer Nadel bekannt, wobei eine über der Nadel zusammenschiebbare Abdeckung vorgesehen ist. Dabei kann der Katheter schon durch kleinere Zugkräfte, welche auf den Katheter wirken, versehentlich wieder herausgezogen werden und liegt relativ ungeschützt an der Einstichstelle offen; siehe Fig. 1 der EP 0 451 040 A1.

Die EP 0 290 176 A1 offenbart eine Vorrichtung zum Einbringen einer Kanüle mit einer Nadel, wobei die Nadel beim Einbringen gegen eine Feder gedrückt werden muss und nach dem Einbringvorgang durch die Federkraft in ein Gehäuse zurückgezogen wird. Auch hier ist die Kanüle nach dem Einbringen relativ ungeschützt und kann leicht versehentlich herausgezogen werden.

Aus der EP 0 615 768 B1 ist eine Vorrichtung zur subkutanen Zufuhr eines Medikaments bekannt. Dabei wird eine Kanüle mit einer Nadel eingebracht, wobei beim Einbringen der Kanüle auch gleichzeitig eine fest mit der Kanüle verbundene Anordnung mit klebender Unterseite auf die Haut aufgebracht wird, was den für einen Anwender häufig unangenehmen Einstichvorgang zusätzlich erschwert.

Bei den bekannten Vorrichtungen ist die Kanüle entweder relativ ungeschützt gegen unbeabsichtigtes Herausziehen, wobei insbesondere schon beim Ausziehen der Nadel aus der Kanüle eine Zugkraft auf die in das Gewebe eingebrachte Kanüle wirkt, oder es muss eine zusätzliche Vorrichtung während des Einstichvorgangs zusammen mit der Kanüle bewegt werden, was die genaue Positionierung erschwert.

Es ist eine Aufgabe der vorliegenden Erfindung eine Vorrichtung vorzuschlagen, welche das Einbringen einer Kanüle verbessert. Allgemein soll ein Infusionsset vorgeschlagen werden, welches Verbesserungen gegenüber dem Stand der Technik aufweist.

Diese Aufgabe wird gelöst durch den Gegenstand des unabhängigen Patenanspruchs. Vorteilhafte Ausführungsformen sind in den abhängigen Ansprüchen aufgeführt.

Ein erfindungsgemässes System zum Verbinden einer Flüssigkeitszufuhr mit einer Kanüle umfasst einen Basiskörper, bestehend aus einem Grundkörper und einem Pflaster, wobei der Grundkörper auf einer ersten Oberfläche des Pflasters aufgebracht ist, und wobei der Grundkörper auf einer ersten Oberfläche des Pflasters aufgebracht ist, und wobei der Grundkörper mindestens eine Öffnung aufweist, und das Pflaster eine zweite, entgegengesetzte, klebende Oberfläche aufweist zum Aufkleben des Basiskörpers auf einer Hautstelle und eine separate Halterung, die fest mit einer Kanüle zum Verabreichen einer Flüssigkeit in eine Gewebe verbunden ist, wobei die Halterung und der Grundkörper dazu konfiguriert sind, bei der Einführung der Kanüle durch den Grundkörper hindurch in ein Gewebe eine Verbindung zu bilden und einen Stecker mit einem Zuführelement als Vorrichtung zum Zuführen einer Flüssigkeit.

Eine Vorrichtung zum Einbringen einer Kanüle in ein Gewebe zum bevorzugt subkutanen oder transkutanen Verabreichen einer Flüssigkeit, weist eine Kanüle auf, welche entweder als Hartkanüle ausgebildet ist, so dass sie ohne zusätzliche Nadel oder ein anderes Einstichelement in das Gewebe z. B. durch Aufnahme von Flüssigkeit eingebracht werden kann, wobei die Kanüle bevorzugt nach dem Einbringen in das Gewebe elastisch bzw. flexibel wird. Es ist jedoch auch möglich eine bekannte Kanüle vorzusehen, welche auf bekannte Art mit z. B. einer Führungsnadel oder einem anderen Einstichelement in das Gewebe eingebracht wird. Weiterhin ist ein Schutzelement vorgesehen, welches die Kanüle vor dem Einbringen in das Gewebe aufnehmen kann, wobei das Schutzelement bevorzugt so ausgebildet ist, dass die in das Gewebe eindringende Spitze der Kanüle oder auch einer zum Einbringen der Kanüle geeigneten Nadel von dem Schutzelement abgedeckt wird, so dass ein Benutzer nicht versehentlich mit dieser Spitze in Kontakt kommen kann und z. B. durch diese Spitze verletzt wird. Das Schutzelement kann die Spitze der Kanüle zum Teil oder vollständig oder sogar die gesamte Kanüle, gegebenenfalls auch eine zum Einbringen der Kanüle vorgesehene Nadel umgeben, um die Schutzfunktion zu gewährleisten. Es ist weiterhin ein Betätigungselement vorgesehen, mit welchem die Kanüle bzw. die Nadel mit Kanüle aus dem Schutzelement herausbewegt werden kann, um z. B. die Kanüle in das Gewebe einzubringen, wobei es im Falle der Verwendung einer Nadel zum Einbringen der Kanüle bevorzugt wird, dass die Nadel nach dem Einbringen der Kanüle durch das Betätigungselement oder auch automatisch, z. B. unter Verwendung eines Federmechanismus, wieder in das Schutzelement zurückbewegt werden kann, um auch nach dem Einbringen der Kanüle die Verletzungsgefahr zu minimieren bzw. auszuschließen. Die Kanüle ist mit einer Halterung verbunden, welche z. B. am distalen, also dem der Kanülenspitze abgewandten Ende vorgesehen sein kann und welche mit der Kanüle bewegt werden kann und beim Einbringen der Kanüle die Kanüle in einer festen Position fixieren kann, indem z. B. diese Halterung mit einem über der Einstichstelle schon vor dem Einstich fest angeordnetem Grundkörper verrastet oder allgemein mit diesem Grundkörper verbunden wird. Wird z. B. eine Nadel nach dem Einbringen der Kanüle aus dieser herausgezogen, so kann aufgrund der mit der Kanüle verbundenen Halterung sichergestellt werden, dass wenn eine Haltekraft an der Halterung anliegt, z. B. durch Verbinden der Halterung mit einem Grundkörper, die Auszugskraft der Nadel nicht unmittelbar auf die Kanüle wirkt, d. h. dass die Kanüle beim Ausziehen nicht in Auszugsrichtung belastet wird. Es ist möglich durch geeignetes Halten bzw. Befestigen der Halterung die Kanüle beim Ausziehen einer Nadel zu entlasten und die Kanüle gegen unbeabsichtigtes Herausziehen zu sichern. Wird eine Kanüle ohne Verwendung einer Nadel eingebracht, so weist die Halterung den Vorteil auf, dass die eingebrachte Kanüle durch die Halterung und gegebenenfalls ein mit der Halterung verbundenes Element gegen unbeabsichtigtes Herausziehen gesichert werden kann. Mit der Vorrichtung ist der Einstichvorgang auch einfach und relativ gefahrlos durchzuführen.

Obwohl in dieser Beschreibung von "Ausschieben" einer Nadel oder Kanüle mit einem "Ausschiebeelement" gesprochen wird, wird angemerkt, dass hierunter auch ein Herausziehen mit einem Zugelement verstanden werden soll, d.h. eine Bewegung kann durch eine Zug- und/oder Druckkraft bzw. eine Zug- und/oder Druckfeder bewirkt werden.

Vorteilhaft ist die mit der Kanüle verbundene Halterung so ausgebildet, dass sie eine Verbindung mit einem anderen Element eingehen kann, bevorzugt mit einem Basiskörper welcher z. B. auf die Haut über einer Einstichstelle aufgeklebt werden kann, wobei dieser Basiskörper vorteilhaft so angeordnet ist, dass die Verbindung mit der Halterung im vollständig oder fast vollständig ausgeschobenen Zustand der Kanüle erfolgt. Diese Verbindung kann eine Rastverbindung oder jede andere geeignete Verbindung sein, wobei z. B. eine oder mehrere Nuten bzw. Vertiefung und/oder Vorsprünge oder Rastlippen an der Halterung vorgesehen sein können, welche eine lösbare oder auch nicht mehr lösbare feste Verbindung der Halterung mit einem geeigneten Element ermöglichen.

Vorzugsweise ist eine Nadel vorgesehen, mit welcher die Kanüle in ein Gewebe eingebracht werden kann, wobei vorteilhaft die Nadel von der Kanüle umgeben wird. Dabei ist es besonders vorteilhaft die Vorrichtung so auszugestalten, dass die Nadel nach Einbringen der Kanüle wieder bevorzugt vollständig zurück in das Schutzelement eingebracht werden kann, z. B. durch eine Bewegung des Betätigungselements und/oder eine Feder, welche z. B. beim Einbringen der Kanüle in das Gewebe und Ausfahren der Nadel aus dem Schutzelement komprimiert wird und eine Kraft erzeugt, welche die Nadel zurück in ihre Ausgangslage in das Schutzelement bringt.

Bevorzugt ist die Vorrichtung so ausgestaltet, dass sie fest oder lösbar mit einem Basiskörper verbunden werden kann und besonders vorteilhaft in einem Ausgangszustand schon mit dem Basiskörper verbunden ist, so dass die Applikation für den Benutzer vereinfacht wird. Der Basiskörper kann z. B. aus einem auf eine Hautstelle aufklebbarem Pflaster und einem darauf angeordneten Grundkörper bestehen, auf welchem die Vorrichtung zum Einbringen der Kanüle angeordnet ist. Bei einer solchen Konfiguration kann die Spitze der Kanüle bzw. der Nadel schon relativ nahe an einer Austrittsöffnung des Basiskörpers angeordnet sein, so dass z. B. nach Aufkleben des Pflasters auf einen Hautbereich die Kanüle bzw. Nadel sofort in die Haut eingebracht werden kann. Dabei kann die Spitze der Kanüle bzw. Nadel auch aus dem Schutzelement herausragen, ohne dass eine Verletzungsgefahr für eine Bedienperson besteht, da die Spitze durch den umgebenden Basiskörper abgeschirmt wird. Die in dem Basiskörper vorgesehene Durchtrittsöffnung für die Spitze der Kanüle bzw. Nadel ist vorteilhaft relativ klein, bevorzugt nur geringfügig größer als der Durchmesser der Kanüle, um ein unbeabsichtigtes Hindurchlangen der Bedienperson durch die Durchtrittsöffnung auszuschließen und somit die Verletzungsgefahr zu minimieren. Nach Einbringen der Kanüle und gegebenenfalls Zurückziehen der Nadel in das Schutzelement kann die Vorrichtung wieder von dem Basiskörper gelöst werden, so dass z. B. eine Flüssigkeitszufuhr mit der Kanüle verbunden werden kann.

Bevorzugt kann die Vorrichtung zum Einbringen der Kanüle mit dem Basiskörper, besonders vorteilhaft mit dem Grundkörper durch ein Verbindungselement, insbesondere eine Rastverbindung verbunden werden, welche bevorzugt auch wieder gelöst werden kann.

Vorzugsweise ist das Schutzelement ein Rahmen, welcher die Kanüle bzw. die Nadel im zurückgezogenen Zustand zumindest teilweise umgibt. Besonders bevorzugt ist das Schutzelement ein Mantel, welcher die Kanüle bzw. Nadel im zurückgezogenen Zustand vollständig umgibt, wobei bevorzugt eine Durchtrittsöffnung in dem Schutzelement vorgesehen ist, durch welches die Kanüle bzw. Nadel aus dem Schutzelement herausbewegt bzw. wieder in das Schutzelement eingebracht werden kann. Diese Durchtrittsöffnung kann offen sein und ist bevorzugt nur geringfügig größer als der Außendurchmesser der Kanüle. Es ist auch möglich die Durchtrittsöffnung durch ein geeignetes Abdeckelement zu verschließen, welches beim Ausfahren der Kanüle bzw. Nadel von der Durchtrittsöffnung wegbewegt werden kann oder auch elastisch ausgebildet ist, so dass z. B. die Kanüle oder Nadel hindurchstechen kann.

Vorteilhaft ist die Vorrichtung so ausgestaltet, dass das Betätigungselement oder auch ein zum Einbringen der Kanüle vorgesehenes Nadelelement im zurückgezogenen Zustand nach Einbringen der Kanüle mit dem Schutzelement verbunden, insbesondere verrastet werden kann, so dass ein versehentliches Wiederausschieben der Nadel aus dem Schutzelement verhindert wird und somit eine unbeabsichtigte Verletzung einer Bedienperson ausgeschlossen werden kann.

Besonders bevorzugt ist die Vorrichtung, insbesondere das Betätigungselement so ausgebildet, dass im ausgeschobenen Zustand der Kanüle ein Lösen der Vorrichtung zum Einbringen der Kanüle von einem mit der Vorrichtung verbundenen Basiskörper verhindert werden kann. Hierzu kann z. B. ein mit dem Betätigungselement verbundenes und verschiebbares Querelement vorgesehen sein, welches im ausgeschobenen Zustand der Kanüle z. B. ein Zusammendrücken von für die Verbindung mit dem Basiskörper vorgesehenen Halteelementen verhindert und somit eine z. B. nur durch Zusammendrücken dieser Halteelemente zu erreichende Loslösung der Vorrichtung von dem Basiskörper ausgeschlossen werden kann. Allgemein kann jede Anordnung bzw. jedes Element verwendet werden, welches eine solche Sicherungsfunktion bieten kann. Hierdurch kann sichergestellt werden, dass nicht versehentlich die Kanülen-Einbringvorrichtung vom Basiskörper gelöst wird, wenn z. B. eine Nadel aus dem Schutzelement ausgefahren ist, was zu Verletzungen einer Bedienperson führen könnte.

Bevorzugt ist in der Halterung ein Dichtelement oder Septum zum Abschließen der Kanülenoberseite bzw. eines Flüssigkeitsraumes vorgesehen, welches z. B. von einer Nadel und/oder einer Flüssigkeitszufuhr durchdrungen werden kann und eine flüssigkeitsdichte Verbindung gewährleisten kann. Ist kein Element in das Septum eingebracht, so kann es den Zugang zur Kanüle oder einem über der Kanüle liegenden Flüssigkeitsraum vollständig verschließen. Geeignete Materialien hierzu sind im Stand der Technik bekannt.

Gemäß einem anderen Aspekt ist ein Basiskörper vorgesehen, welcher z. B. aus einem auf eine Hautstelle aufklebbarem Pflaster und einem darauf angeordneten Grundkörper besteht, wobei der Basiskörper bzw. der Grundkörper mindestens ein bevorzugt lösbares Verbindungselement aufweist, an welchem die oben beschriebenen Vorrichtung zum Einbringen einer Kanüle angebracht werden kann und an welchem eine Vorrichtung zum Zuführen eines Fluids bzw. einer Flüssigkeit angebracht werden kann, so dass wenn der Basiskörper über einer Einbringstelle der Kanüle angebracht ist, an dem Basiskörper sowohl die Vorrichtung zum Einbringen der Kanüle, als auch eine Vorrichtung zum Zuführen eines Fluids zusammen oder nacheinander an den gleichen oder verschiedenen Verbindungselementen angebracht werden können. Dabei kann bevorzugt im Ausgangszustand die Vorrichtung zum Einbringen der Kanüle schon fest und lösbar mit dem Basiskörper verbunden sein, so dass der Basiskörper mit der Einbringvorrichtung für die Kanüle zusammen über einer Einbringstelle der Kanüle angebracht werden kann, wobei die Einbringvorrichtung nach erfolgtem Einbringen der Kanüle von dem Basiskörper wieder abgelöst werden kann. Es ist auch möglich den Basiskörper erst vor dem Einbringen der Kanüle mit der Kanülen-Einbringvorrichtung zu verbinden.

Vorteilhaft dient das mindestens eine am Basiskörper vorgesehene Verbindungselement sowohl zur Verbindung mit der Einbringvorrichtung für die Kanüle, als auch zur Verbindung mit der Vorrichtung zum Zuführen eines Fluids, so dass z. B. nach Ablösen der Einbringvorrichtung für die Kanüle die Vorrichtung zum Zuführen des Fluids an dem bzw. den gleichen Verbindungselementen angebracht werden kann, welches bzw. welche zuvor zum Befestigen der Einbringvorrichtung für die Kanüle gedient haben.

Bevorzugt ist das mindestens eine am Basiskörper vorgesehene Verbindungselement ein Element, welches eine Rastverbindung ermöglicht, also insbesondere eine Nut und/oder eine Rastlippe bzw. Rastnase mit welcher eine Rastverbindung mit der Einbringvorrichtung für die Kanüle und/oder der Fluidzuführvorrichtung hergestellt werden können.

Gemäß einem Aspekt der vorliegenden Erfindung wird ein System vorgeschlagen, mit welchem eine Flüssigkeitszufuhr mit einer Kanüle verbunden werden kann, wobei ein Grundkörper vorgesehen ist, welcher eine Kanüle aufweist, die bevorzugt schon in ein Gewebe eingebracht ist, z. B. unter Verwendung der oben beschriebenen Vorrichtung. Der Grundkörper hat mindestens eine Öffnung, welche mit der Kanüle bzw. dem Kanülenhohlraum in Verbindung steht. Zur Zufuhr der Flüssigkeit ist ein Stecker mit einem Zuführelement vorgesehen, welches in die Öffnung des Grundkörpers eingebracht werden kann, so dass die Flüssigkeit über das Zuführelement durch die Öffnung des Grundkörpers in den Kanülenhohlraum und somit in das Gewebe geleitet werden kann. Erfindungsgemäß kann der Stecker an einem Anlagepunkt des Grundkörpers angelegt und so um den Anlagepunkt geklappt werden, dass das Zuführelement des Steckers in die Öffnung des Grundkörpers geführt wird. Ein solches Verbinden bzw. Konnektieren des Steckers einer Flüssigkeitszufuhr mit der Kanüle ist vorteilhaft, da keine exakte Positionierung am Anfang des Verbindungsvorganges erforderlich ist, d. h. dass z. B. in ihrer körperlichen Leistungsfähigkeit eingeschränkte Benutzer den Stecker relativ einfach an einem Anlagepunkt des Grundkörpers anlegen können, wobei der Anlagepunkt kein Punkt im geometrischen Sinne sein muss, sondern auch als Anlagekante mit geradem oder auch gebogenem bzw. abgewinkeltem Verlauf oder als Anlagefläche ausgebildet sein kann. Wird ein solcher Stecker z. B. mit einer Anlagekante bzw. einem geeigneten Vorsprung des Steckers an einer Anlagekante des Grundkörpers angelegt, was noch nicht mit großer Genauigkeit bezüglich der Anlageposition erfolgen muss, so kann z. B. durch geeignete Führungen der Stecker beim Klappen um diese Anlagekante allmählich in die exakte Position gebracht werden, so dass das Zuführelement bei vollständig niedergeklapptem Stecker exakt in die Öffnung des Grundkörpers eingebracht werden kann. Diese Art der Verbindung eines Steckers mit dem Grundkörper ist somit bezüglich des Ansatzes des Steckers am Grundkörper relativ fehlertolerant und ist insbesondere dann von großem Vorteil, wenn die mit dem Grundkörper verbundene Kanüle wie oben beschrieben durch eine bereits fest mit dem Grundkörper verbundene Einbringvorrichtung für die Kanüle eingeführt wurde. In diesem Fall muss die Einbringvorrichtung für die Kanüle vom Benutzer nach dem Einbringen der Kanüle lediglich von dem Grundkörper gelöst werden, was keinen genauen Positionierungsvorgang erfordert, so dass der einzige Positionierungsvorgang, welcher vom Benutzer ausgeführt werden muss, das fehlertolerante Anbringen des Steckers an dem Anlagepunkt des Grundkörpers ist, wobei bevorzugt über eine geeignete Führung beim Klappen des Steckers das Zuführelement positionsgenau in die Öffnung des Grundkörpers gebracht wird.

Bevorzugt ist mindestens ein Führungselement am Grundkörper und/oder am Stecker vorgesehen, um den Stecker während des Klappvorgangs nach dem Anlegen an dem Anlagepunkt bzw. einer Anlagekante zu führen. Als Führungselement kann z. B. eine sich in der Breite verjüngende Rille vorgesehen sein, in welche ein Vorsprung eingreift, so dass der Vorsprung bei dem Klappvorgang entlang der Rille in Richtung auf das schmalere Ende geführt wird, wodurch eine genaue Positionierung des Steckers relativ zum Grundkörper erfolgen kann. Sowohl die Rille, als auch der Stecker können dabei an dem Grundkörper und/oder dem Stecker vorgesehen sein. Weiterhin ist es auch denkbar, seitliche Führungen an den Stecker und/oder dem Grundkörper vorzusehen, welche trichterförmig ausgebildet sind, um so die gewünschte Positionierung des Steckers relativ zu dem Grundkörper zu erzielen. Allgemein ist jedoch jede Anordnung geeignet, welche es ermöglicht, dass der an mindestens einem Anlagepunkt anliegende Stecker bei einem Klappvorgang geführt und dabei im niedergeklappten Zustand genau positioniert werden kann.
Bevorzugt ist der Stecker so ausgebildet, dass er mit dem Anlagepunkt bzw. einer Anlagekante des Grundkörpers verhaken kann. Unter Verhaken wird im Sinne der Erfindung verstanden, dass der Stecker an einem Punkt oder mehreren Punkten, Kanten oder Flächen des Grundkörpers anliegt und so eine lose Verbindung zwischen Stecker und Grundkörper geschaffen wird, welche ein Klappen bzw. eine Drehbewegung um diese Verbindung ermöglichen. Dabei soll bevorzugt mindestens ein Freiheitsgrad in der Bewegung des Steckers relativ zum Grundkörper eingeschränkt werden, so dass nach Einhaken des Steckers in den Grundkörper eine erste Grobpositionierung des Steckers in Bezug auf den Grundkörper erfolgt.

Besonders bevorzugt kann der Stecker mit dem Grundkörper verbunden werden, z. B. verrasten, wobei hierzu geeignete Nuten, Rastnasen oder Ähnliches vorgesehen sein können. Die Verbindung bzw. Rastverbindung kann lösbar oder unlösbar ausgestaltet sein.

Vorzugsweise weist ein Grundkörper ein drehbar gelagertes Drehteil auf, welches bevorzugt fest mit dem Grundkörper verbunden ist. Das Drehteil hat eine bevorzugt durch ein Dichtungselement abgeschlossene Öffnung, welche in einer ersten Position des Drehteils das Einführen einer Kanüle z. B. mit einer Nadel ermöglicht und in einer zweiten gedrehten Position das Einführen eines Zuführelements zum Zuführen von Flüssigkeit ermöglicht. Das Vorsehen eines Drehteils am Grundkörper bringt den Vorteil mit sich, dass z. B. bei Ausrichtung der Öffnung des Drehteiles nach oben, also in eine Richtung bei welcher die Öffnung auf einer Verlängerung der gewünschten Position der einzubringenden Kanüle liegt, die Kanüle durch die Öffnung des Drehteils und durch den Grundkörper hindurch direkt in das Gewebe eingebracht werden kann. Nach erfolgtem Einbringen der Kanüle und gegebenenfalls dem Loslösen der Einbringvorrichtung der Kanüle kann das Drehteil gedreht werden, so dass seitlich bzw. bei nicht nach oben weisender Öffnung des Drehteils ein Zuführelement zum Zuführen von Flüssigkeit angeschlossen werden kann. Somit kann auf einfache Weise eine Kanüle eingebracht werden und eine Flüssigkeitszufuhr seitlich angeschlossen werden, wodurch die Gesamthöhe der Vorrichtung bei eingebrachter Kanüle und angeschlossener Flüssigkeitszufuhr gering bleibt. Die Verbindung der Kanüle mit der Öffnung des Drehteils kann z. B. durch ein flexibles Schlauchelement oder eine andere geeignete Vorrichtung erfolgen, welche eine sichere Verbindung im gedrehten Zustand des Drehteils ermöglicht.

Vorzugsweiseweist eine Vorrichtung zum Zuführen einer Flüssigkeit über eine Kanüle in ein Gewebe nur ein einziges Dichtungselement auf, welches zur Abdichtung eines Flüssigkeitsraumes dient und von einer Kanüle und/oder einer Nadel durchstochen werden kann, wenn die Kanüle in das Gewebe eingebracht werden soll und von einem Zuführelement durchdrungen werden kann, wenn dem Flüssigkeitsraum eine Flüssigkeit zugeführt werden soll. Ergänzend können natürlich auch weitere Dichtungselemente vorgesehen sein.

Vorzugsweise kann eine Flüssigkeit über eine Kanüle einem Gewebe zugeführt werden, wobei eine Kanüle, gegebenenfalls mit Nadel, durch ein Dichtungselement gestochen wird, um die Kanüle in das Gewebe einzubringen. Ist die Kanüle eingebracht, so wird ein Zuführelement, gegebenenfalls nach Entfernen der Nadel, durch das Dichtungselement eingebracht, um über das Zuführelement durch das Dichtungselement und die Kanüle hindurch eine Flüssigkeit in das Gewebe einzubringen.

Vorzugsweise wird eine Vorrichtung zum Einbringen einer Kanüle in Gewebe vorgeschlagen, wobei eine Kanülenausschubvorrichtung zum Ausschieben der Kanüle und Einbringen der Kanüle in Gewebe vorgesehen ist. Ein Rückzugelement ist mit der Kanülenausschubvorrichtung gekoppelt, um die Kanülenausschubvorrichtung nach dem Ausschieben der Kanüle wieder zurückzuziehen. Das Rückzugelement ist bevorzugt eine Feder, welche beispielsweise so vorgespannt sein kann, dass die in der Feder gespeicherte Energie bzw. Kraft ausreicht die Kanülenausschubvorrichtung aus dem ausgeschobenen Zustand wieder zurückzuziehen, wobei ein vollständiges Zurückziehen in den Ausgangszustand möglich aber nicht erforderlich ist. Somit kann die Applikation einer Kanüle automatisiert und damit vereinfacht werden. Durch geeignete Wahl der Parameter des Rückzugelements, wie z.B. einer Federlänge und einer Federkonstante, kann der Rückzugsvorgang der Kanülenausschubvorrichtung sicher durchgeführt werden, d.h. es steht bei richtiger Wahl dieser Parameter immer eine ausreichende Kraft zur Verfügung, um das Rückzugelement sicher zurückzuziehen, ohne dass ein Benutzer manuell eine große Kraft aufwenden muss.

Bevorzugt ist die Kanülenausschubvorrichtung eine Führungsnadel oder ein anderes die Kanüle tragende Element. So kann z. B. eine Kanüle ohne Führungsnadel mittels einer geeigneten Kanülenhalterung eingebracht werden.

Als Rückzugelement ist bevorzugt eine Feder vorgesehen, wobei auch andere Energie- oder Kraft speichernde Elemente eingesetzt werden können, um das Rückzugelement nach Einbringen der Kanüle wieder zurückzuziehen.

Bevorzugt ist ein Auslöseelement für das Rückzugelement vorgesehen, welches z. B. manuell bedient werden kann oder bei einem bestimmten Zustand der Kanüleneinbringvorrichtung automatisch ausgelöst wird. Als manuelles Auslöseelement kann z. B. ein Druckknopf oder ein geeignetes anderes Schalt- oder Schiebeelement vorgesehen sein, mit welchem eine Sicherung des Rückzugelements entsichert werden kann. Als Sicherung kann z. B. ein das Rückzugelement in Rückzugrichtung blockierendes Halteelement vorgesehen sein, welches bei Auslösen der Sicherung, z. B. dem Drücken eines Druckknopfes, so verschoben wird, dass über das Rückzugelement eine Rückzugkraft an die Kanülenausschubvorrichtung angelegt wird, um diese zurückzuziehen. Das Halteelement kann z. B. ein seitlich verschiebbares Element, wie z. B. ein Stift oder eine Kante sein und kann z. B. auch über einen Kipp- oder Klappmechanismus bewegt werden.

Vorteilhaft kann das Auslöseelement für das Rückzugelement automatisch bei einem bestimmten Zustand der Kanüleneinbringvorrichtung ausgelöst werden. Zum Beispiel kann ein Mechanismus vorgesehen sein, welcher den Rückzugsvorgang der Kanülenausschubvorrichtung automatisch einleitet, wenn z. B. die Kanüleneinbringvorrichtung von einem Grundkörper abgenommen wird. Hierzu kann z. B. eine Nocke am Grundkörper vorgesehen sein, welche beim Abnehmen der Kanüleneinbringvorrichtung vom Grundkörper automatisch einen Auslösemechanismus für das Rückzugelement betätigt.

Vorteilhaft kann auch ein Ausschubelement, z. B. eine Feder vorgesehen sein, welche eine ausreichende Kraft erzeugen kann, um die Kanüle in das Gewebe einzubringen. Das Ausschubelement kann wie oben für das Rückzugelement beschrieben gesichert sein und z. B. ebenfalls durch einen Druckknopf ausgelöst werden. Bei Vorsehen sowohl eines Rückzugelements, als auch eines Ausschubelements kann eine vollautomatische Kanüleneinbringvorrichtung geschaffen werden, da ein Benutzer weder zum Einbringen einer Kanüle, noch zum Zurückziehen einer Kanülenausschubvorrichtung aktiv Kraft aufwenden muss. Das Einbringen einer Kanüle z. B. mit einer Führungsnadel und das Zurückziehen der Führungsnadel wird somit voll automatisiert, so dass die Gefahr einer falschen Anwendung durch Benutzer verringert wird.

Die Vorrichtung zum Einbringen einer Kanüle in Gewebe kann vorteilhaft so ausgestaltet sein, dass ein einziges energiespeicherndes Element, wie z. B. eine Feder oder auch eine Mehrzahl von zusammenwirkenden energiespeichernden Elementen als eine energiespeichemde Einheit eingesetzt werden, um eine Kanülenausschubvorrichtung oder Nadel, bevorzugt nach dem geeigneten Positionieren, automatisch, d.h. ohne Zuführen von externer Energie, so zu bewegen, dass die Kanüle in das Gewebe eingebracht wird und die Kanülenausschubvorrichtung anschließend ebenfalls ohne externe Kraft- oder Energiezufuhr automatisch wieder in das Gewebe eingebrachten Kanüle herausgezogen wird, so dass ein Benutzer, außer zum Auslösen der jeweiligen Ausschub- und Rückzieh-Vorgänge, im wesentlichen keine Kraft aufwenden muss. Dabei ist es vorteilhaft bei Verwendung einer einzigen energiespeichernden Vorrichtung, wie beispielsweise einer Druckfeder, die Feder im Ausgangszustand, d.h. vor Einbringen der Kanüle in Gewebe, in einem gespannten Zustand so anzuordnen, dass bei einer ersten Teilentspannung oder Teilausdehnung der Feder in eine erste Richtung, beispielsweise zum Ausschieben der Kanülenausschubvorrichtung oder Nadel aus der Kanüleneinbringvorrichtung nach unten, die Kanülenausschubvorrichtung oder Nadel soweit aus der Kanüleneinbringvorrichtung herausbewegt wird, dass die Kanüle oder eine Kanülenbaugruppe wie gewünscht in oder auf einem Gewebe plaziert werden können, wobei bei einer zweiten Teilausdehnung der Feder in eine zweite Richtung, bevorzugt entgegengesetzt zur ersten Richtung, die Kanülenausschubvorrichtung oder Nadel wieder zurückgezogen wird, so dass die Kanüle oder die Kanülenbaugruppe im Gewebe verbleiben kann und die Kanüleneinbringvorrichtung mit bevorzugt vollständig eingezogener Kanülenausschubvorrichtung oder Nadel abgenommen werden kann. Statt einer Feder, welche beispielsweise aus Metall oder Kunststoff gefertigt sein kann, können als Energiespeicher auch andere Elemente verwendet werden, welche beispielsweise Druckluft speichern, auf elektrischen, magnetischen oder anderen Prinzipien basieren.

Bevorzugt ist ein einziges Auslöseelement, wie beispielsweise ein Knopf, Schalter, Klappmechanismus, Schiebemechanismus, Rastmechanismus, Drehmechanismus, Drehknopf oder Hebel vorgesehen, mit welchem je nach Stellung der Ausschub-Vorgang und der Rückzieh-Vorgang der Kanülenausschubvorrichtung oder Nadel ausgelöst werden können. Beispielsweise kann ein Knopf in Form eines einschiebbaren oder drückbaren Elements vorgesehen sein, welcher z. B. nach Durchführen eines Entsicherungs-Vorganges eine erste Teilstrecke gedrückt wird, um den Ausschub-Vorgang auszulösen und in der gleichen Richtung eine zweite Teilstrecke mit gleicher oder unterschiedlicher Länge gedrückt wird, um den Rückzugvorgang auszulösen. Alternativ ist es auch möglich, dass das eine Auslöseelement zum Auslösen des Ausschubvorgangs in eine erste Richtung betätigt wird und zum Auslösen des Rückzug-Vorgangs der Kanülenausschubvorrichtung oder Nadel in eine zweite Richtung bewegt wird, welche von der ersten Richtung verschieden ist, wie z. B. eine Bewegung in die entgegengesetzte Richtung. Somit kann mit einem einzigen Auslöseelement, wie beispielsweise einem Druckknopf, durch Drücken des Druckknopfes z. B. in eine einzige Richtung nacheinander zunächst das Einbringen der Kanüle in Gewebe durch die Kanülenausschubvorrichtung oder Nadel und anschließend das Rückziehen der Nadel aus der eingebrachten Kanüle oder Kanülenbaugruppe veranlasst werden, wodurch die Kanüleneinbringvorrichtung sehr einfach bedient werden kann.

Allgemein kann ein Auslöseelement auch als ein Drehmechanismus oder Drehknopf ausgebildet sein, wobei durch eine Drehung ein Ausschub-Vorgang ausgelöst werden kann und bei einem Weiterdrehen in die gleiche oder alternativ in die entgegengesetzte Richtung ein Rückzug-Vorgang ausgelöst wird. Es sind auch Kombinationen unterschiedlicher Auslöseelemente möglich, um z.B. mit einem Druckknopf oder Schalter einen Ausschub- Vorgang auszulösen und z.B. mit einem Drehknopf einen Rückzug-Vorgang auszulösen. Vorteilhaft wird ein Auslöseelement für das Rückziehen erst nach erfolgtem Ausschieben freigegeben.

Vorteilhaft kann mindestens ein Sicherungselement an der Kanüleneinbringvorrichtung vorgesehen sein, welches ein unbeabsichtigtes Betätigen eines Auslöseelements verhindert. Ein solches Sicherungselement ist beispielsweise so ausgestaltet, dass es von der Kanüleneinbringvorrichtung abgenommen oder an der Kanüleneinbringvorrichtung in einen entsicherten Zustand gebracht werden muss, um überhaupt das Auslöseelement betätigen zu können. Beispielsweise kann das Sicherungselement als eine Sicherungskappe ausgestaltet sein, welche das Auslöseelement zumindest teilweise und bevorzugt in etwa vollständig umgibt und vor unbeabsichtigter Berührung und somit ungewünschter Auslösung schützt. Weiterhin kann das Sicherungselement auch als eine Vorrichtung zum Sperren oder Arretieren des Auslöseelements ausgestaltet sein, welche beispielsweise erst verschoben werden muss, um das Auslöseelement betätigen zu können. Dabei ist es vorteilhaft das Sicherungselement so auszugestalten, dass es nicht einfach z. B. durch eine unbeabsichtigte Berührung oder einen Stoß bewegt werden kann, sondern beispielsweise nur durch eine Druckbewegung zwischen zwei Fingern in einen Zustand gebracht werden kann, in welchem das Auslöseelement betätigt werden kann. Dabei kann das Sicherungselement so ausgestaltet sein, dass es entweder einen konstanten Druck oder Zug benötigt, um das Auslöseelement in den entsicherten Zustand zu versetzen, oder dass es nach Durchführen eines Entsicherungsvorgangs in dem entsicherten Zustand bleibt und beispielsweise einrastet, so dass nach dem Entsichern das Auslöseelement betätigt werden kann.

Die Kanüleneinbringvorrichtung kann als Einwegvorrichtung ausgebildet sein, wobei das darin enthaltene Rückzugelement und/oder Ausschubelement schon vorgespannt sind, um ein automatisches Einbringen der Kanüle und/oder ein automatisches Rückziehen der Kanülenausschubvorrichtung zu bewirken. Die Kanüleneinbringvorrichtung kann jedoch auch als Mehrweg-Kanüleneinbringvorrichtung ausgebildet sein, wobei das Rückzugelement und/oder das Ausschubelement als ladbar bzw. spannbar ausgebildet sind. So kann z. B. ein Mechanismus vorgesehen sein, um eine Rückzugfeder und/oder eine Ausschubfeder nach erfolgtem Einbringen einer Kanüle und/oder Zurückziehen einer Kanülenausschubvorrichtung wieder zu spannen, so dass die Kanüleneinbringvorrichtung zum Einbringen einer weiteren Kanüle verwendet werden kann.

Allgemein kann die Kanüleneinbringvorrichtung voll automatisch ausgestaltet werden, also sowohl ein Ausschubelement für die Kanüle, als auch ein Rückzugelement vorgesehen sein. Alternativ ist es auch möglich nur eines dieser beiden Elemente vorzusehen, um eine halbautomatische Kanüleneinbringvorrichtung zu schaffen, wobei dann der jeweils andere Vorgang manuell durchgeführt werden muss. Es ist z. B. möglich nur das Einbringen der Kanüle durch Vorsehen eines Ausschubelements zu automatisieren, wobei das Zurückziehen der Kanülenausschubvorrichtung dann manuell durchgeführt werden muss.

Alle beschriebenen Ausführungsformen eines Infusionssets bzw. einer Kanüleneinbringvorrichtung und/oder einer Flüssigkeitszuführvorrichtung können abweichend von den voranstehend beispielhaft beschriebenen Rastverbindungen zum Verbinden der jeweiligen Vorrichtungen mit einem Grundkörper auch eine Dreh- oder Schraubverbindung aufweisen, welche bevorzugt in einer oder mehreren Stellungen verrasten können, so dass die jeweiligen Vorrichtungen durch Drehen miteinander verbunden bzw. voneinander gelöst werden können und auf die beschriebenen Rastvorrichtungen verzichtet werden kann.

Die oben beschriebenen Vorrichtungen gemäß den einzelnen Aspekten können sowohl unabhängig voneinander, als auch in Kombination mit Elementen gemäß anderen Aspekten verwendet werden.

Die Erfindung wird nachfolgend anhand bevorzugter Ausführungsbeispiele unter Bezugnahme auf die Zeichnungen beschrieben werden. Es zeigen:
- Figur 1: eine erste Ausführungsform eines Infusionssets einer an einem Basiskörper angebrachten Vorrichtung zum Einbringen der Kanüle;
- Figur 2: die Vorrichtung nach Figur 1, wobei die Kanüleneinbringvorrichtung vom Basiskörper losgelöst ist;
- Figur 3: eine Teilschnittansicht des Basiskörpers mit eingebrachter Kanüle;
- Figur 4: eine Querschnittsansicht einer Flüssigkeitszuführvorrichtung;
- Figur 5: eine Teilquerschnittsansicht der in den Basiskörper einzubringenden Flüssigkeitszuführvorrichtung;
- Figur 6: die Anordnung nach Figur 5 mit an dem Basiskörper angesetztem Stecker der Flüssigkeitszuführvorrichtung;
- Figur 7: die Anordnung von Figur 6 aus einem anderem Blickwinkel;
- Figur 8: eine alternative Ausführungsform mit Drehteil;
- Figur 9: eine Ausführungsform einer automatischen Kanüleneinbringvorrichtung im Ausgangszustand;
- Figur 10: die in Figur 9 gezeigte Kanüleneinbringvorrichtung nach Einbringen der Kanüle;
- Figur 11: die in Figur 10 gezeigte Kanüleneinbringvorrichtung nach Rückziehen der Führungsnadel;
- Figur 12: die in Figur 11 gezeigte Kanüleneinbringvorrichtung nach Abtrennung von dem Grundkörper;
- Figur 13: eine erste Ausführungsform einer automatischen Kanüleneinbring- und Rückzugsvorrichtung im Ausgangszustand;
- Figur 14: die Vorrichtung von Figur 13 nach Einbringen der Kanüle;
- Figur 15: die in Figur 14 gezeigte Vorrichtung nach Rückziehen der Führungsnadel;
- Figur 16: eine zweite Ausführungsform einer automatischen Kanüleneinbring- und Rückzugsvorrichtung im Ausgangszustand;
- Figur 17: die in Figur 16 gezeigte Vorrichtung nach Einbringen der Kanüle; und
- Figur 18: die in Figur 17 gezeigte Vorrichtung nach Rückziehen der Führungsnadel.

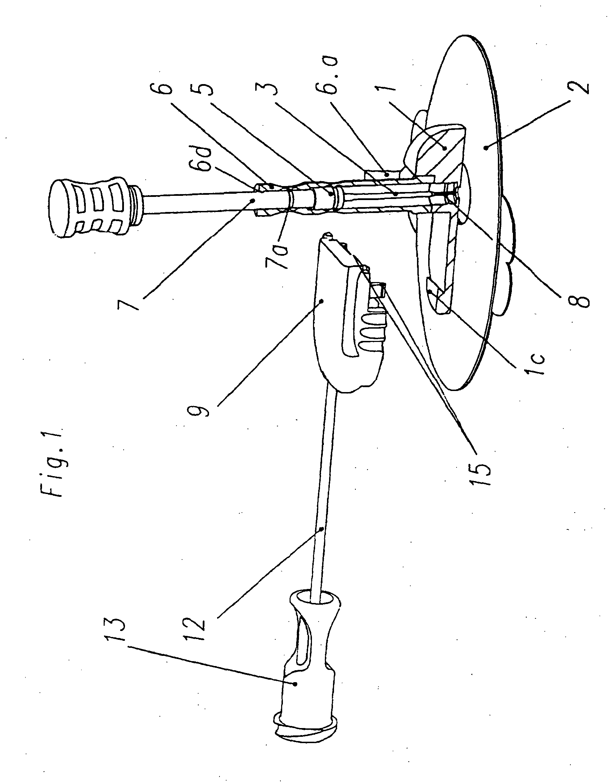

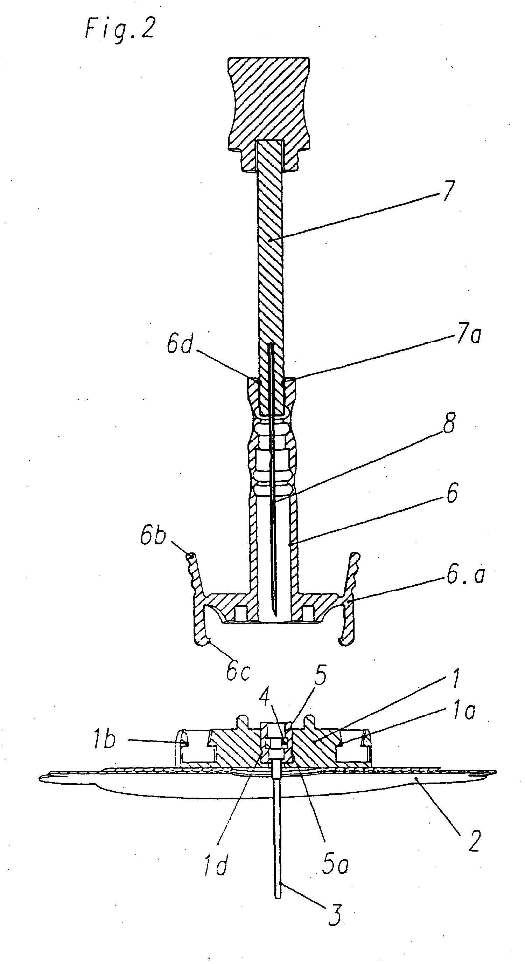

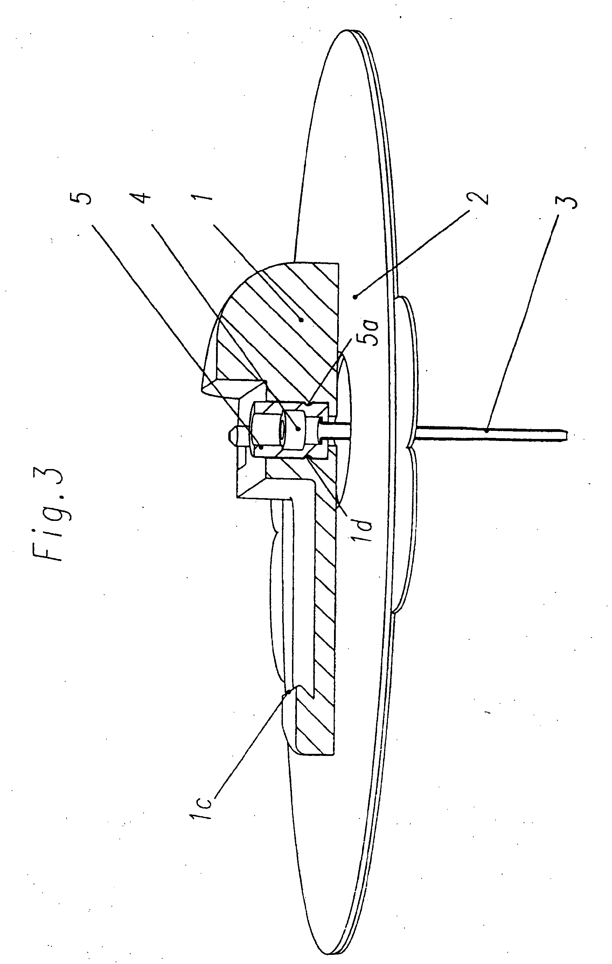

Figur 1 zeigt ein Infusionsset mit fest auf dem Basiskörper 1, 2 vormontierter Kanülen-Einbringvorrichtung 3 - 8. Der Basiskörper besteht aus einem Grundkörper 1 und einem Pflaster 2, welches eine untere klebende Oberfläche aufweist, um den Basiskörper über einer Injektionsstelle aufkleben zu können. Auf der Oberseite des Pflasters 2 ist der Grundkörper 1 angeordnet; z. B. aufgeklebt, welcher fest durch den Halter 6a mit der Kanülen-Einbringvorrichtung 3 - 8 verbunden ist. Die Kanülen-Einbringvorrichtung weist eine Führungsnadel 8 auf, welche durch die Kanüle 3 hindurchgeführt ist und mit welcher die Kanüle 3 aus der Unterseite des Grundkörpers 1 heraus in ein Gewebe eingebracht werden kann. An der Oberseite der Kanüle 3, d. h. der der Spitze der Führungsnadel 8 abgewandten Seite der Kanüle 3 ist eine Halterung 5 fest mit der Kanüle 3 verbunden, wobei in der Halterung 5, wie in Figur 3 gezeigt, ein Dichtelement bzw. Septum 4 vorgesehen ist. Die Führungsnadel 8 und die Kanüle 3 sind von dem als Schutzelement dienenden Führungselement 6 umgeben, so dass einerseits keine Verletzungsgefahr einer Bedienperson besteht, da die Spitze der Führungsnadel 8 nicht aus der gezeigten Anordnung herausragt und noch innerhalb des Grundkörpers 1 bzw. des Führungselements 6 angeordnet ist. Andererseits wird durch die in Figur 1 gezeigte Anordnung eine Kontamination der Führungsnadel 8 und der Kanüle 3 vor Einbringen der Kanüle 3 in Gewebe weitgehend verhindert, da ein direkter Kontakt der Kanüle 3 und Führungsnadel 8 zur Umgebung durch das Führungselement 6 und den Grundkörper 1 verhindert wird. Die Führungsnadel 8 ist, wie in Figur 2 gezeigt, fest mit dem Betätigungselement 7 verbunden und kann durch Druck auf das Betätigungselement 7 nach unten aus dem Grundkörper 1 ausgeschoben werden, um die Kanüle 3 in ein Gewebe einzubringen.

Figur 2 zeigt die Anordnung von Figur 1 nach dem Einführen der Kanüle 3 und dem Lösen der Verbindung zwischen Kanülen-Einbringvorrichtung und Basiskörper. Durch Drücken des Betätigungselements 7 nach unten wurde die Führungsnadel 8 zusammen mit der Kanüle 3 nach unten verschoben und die Kanüle 3 in ein nicht dargestelltes unter dem Pflaster 2 liegendes Gewebe eingebracht. Die an der Oberseite der Kanüle 3 fest mit der Kanüle 3 verbundene Halterung 5 mit innenliegendem Dichtelement 4 wurde dabei so weit in den Grundkörper 1 eingeschoben, bis ein ringförmiger Vorsprung 1d des Grundkörpers 1 in eine um die Halterung 5 umlaufende Rille 5a eingreift und somit eine Verrastung der Halterung 5 in den Grundkörper 1 bewirkt. Nach erfolgter Verrastung der Halterung 5 im Grundkörper 1 kann durch eine Bewegung des Betätigungselements 7 nach oben die Führungsnadel 8 aus der Kanüle 3 herausgezogen werden, ohne dass bei diesem Ausziehvorgang der Führungsnadel 8 aus der Kanüle 3 eine allzu große Kraft auf die eingebrachte Kanüle 3 wirkt. Die Verrastung der Halterung 5 in den Grundkörper 1 bewirkt, dass bei einer relativ zum, beim Ausziehvorgang noch mit dem Grundkörper 1 verbundenen, Führungselement 6 nach oben wirkenden Kraft auf die Führungsnadel 8 die Halterung 5 fest in dem Grundkörper 1 verankert ist und somit eine Zugentlastung der Kanüle 3 beim Ausziehvorgang der Nadel 8 ermöglicht wird.

Die Halterung 5 kann z.B. an der Außenfläche, welche im Inneren des Führungselements 6 geführt wird eine Oberflächenstruktur oder ein oder mehrere nach außen abstehende Elemente, aufweisen, welche eine Bewegung der Halterung 5 in Ausschubrichtung ermöglichen, jedoch eine Bewegung in Rückzugrichtung verhindern oder erschweren, so dass eine Fehlbedienung verhindert werden kann. Entsprechend können an der Innenseite des Führungselements 6 korrespondierende Strukturen oder Elemente vorgesehen sein, um eine Bewegung nur in eine vorgegebene Richtung zu ermöglichen.

Werden die Halter 6a des Führungselements 6 durch Zusammendrücken der Betätigungselemente 6b so bewegt, dass die Rastnasen 6c des Führungselements 6 nicht mehr in die Rastnasen 1a des Grundkörpers 1 eingreifen, so kann die Kanülen-Einbringvorrichtung von dem Grundkörper 1 abgelöst werden. Das Betätigungselement 7 ist in seiner äußersten oberen Stellung durch eine im unteren Bereicht des Betätigungselements 7 umlaufende Nut 7a fest mit einem umlaufende Vorsprung 6d des Führungselements 6 verrastet und stellt somit sicher, dass nach Diskonnektieren der Kanülen-Einbringvorrichtung die Führungsnadel 8 nicht mehr unbeabsichtigt aus dem Führungselement 6 ausgeschoben werden kann. Wie aus Figur 1 ersichtlich, ist im vormontierten Grundzustand der Kanülen-Einbringvorrichtung auf dem Grundkörper 1 das Betätigungselement 7 so weit in das Führungselement 6 eingefahren, dass die umlaufende Nut 7a des Betätigungselements 7 unterhalb des umlaufenden Vorsprunges 6d positioniert ist, so dass eine Verrastung des Betätigungselements 7 mit dem Führungselement 6 erst nach dem Einbringen der Kanüle 3 erfolgt.

Figur 3 zeigt im Teilschnitt die eingeführte Kanüle 3 mit der im Grundkörper 1 verrasteten Halterung 5, in welcher ein Dichtelement 4 angeordnet ist.

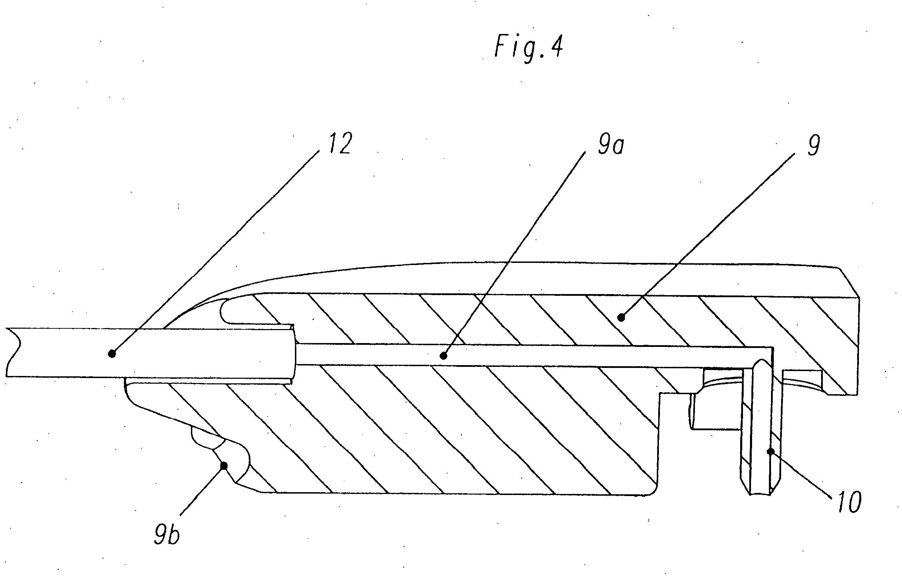

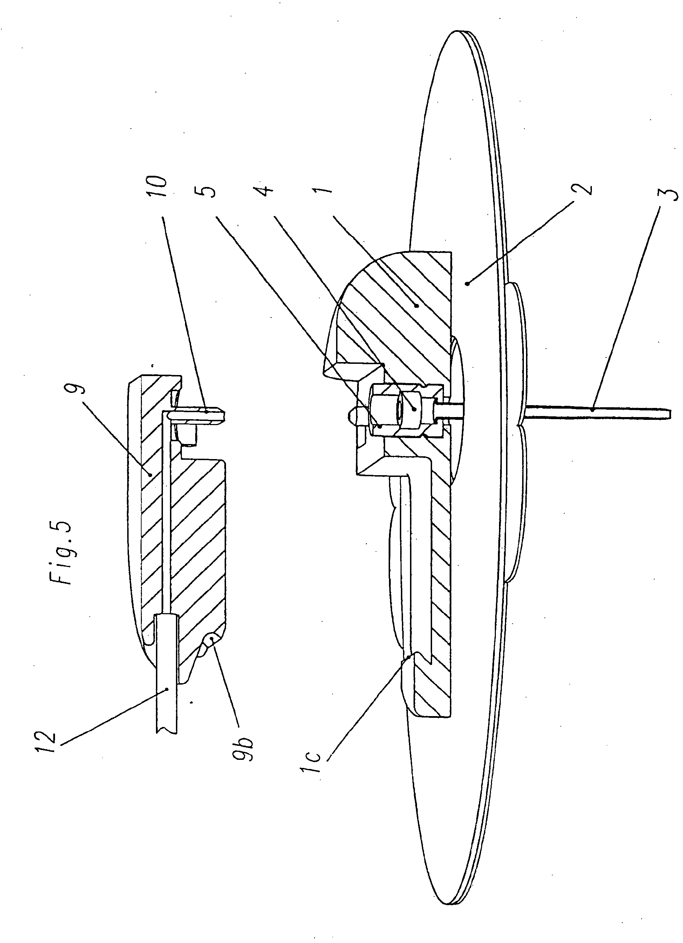

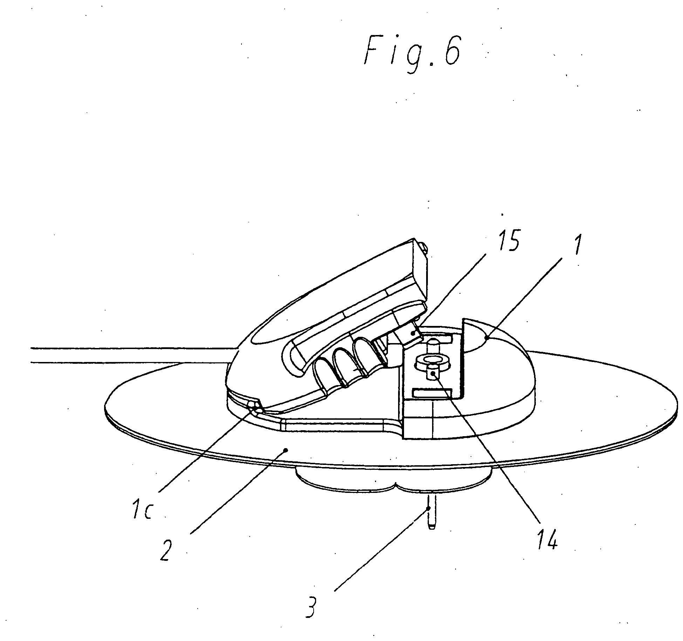

Figur 4 zeigt ein Schnittbild des in Figur 1 gezeigten Steckers 9 der Flüssigkeitszuführvorrichtung. Das Kupplungsteil 13 des Schlauches 12 wird mit einer Flüssigkeitsfördereinrichtung (nicht gezeigt) verbunden. Anschließend wird der gesamte Flüssigkeitsraum des Kupplungsteils 13, des Schlauches 12, sowie des Steckers 9 geflutet. Der Stecker weist eine Steckerkanüle 10 auf, welche durch den im Stecker 9 verlaufenden Kanal 9a mit dem Schlauch 12 verbunden ist. Der Stecker 9 wird in eine Position über dem Grundkörper 1 gebracht, wie in Figur 5 gezeigt und mit der an der hinteren unteren Seite des Steckers 9 verlaufenden Kante 9b in Kontakt mit der Kante 1b des Grundkörpers 1 gebracht. Hierbei wird der Stecker 9 bevorzugt leicht nach oben gekippt, wie in Figur 6 gezeigt, so dass die vom Stecker 9 vorstehende Kante 9b in den durch die Oberseite des Grundkörpers 1 und die vorspringende Kante 1c des Grundkörpers 1 definierte Spalte eingebracht wird. Somit kann der Stecker 9 relativ einfach und bedienungsfreundlich in eine erste Anlageposition zu dem Grundkörper 1 gebracht werden. Greift die Kante 9b des Steckers 9 in dem durch die Kante 1c des Grundkörpers 1 definierten Spalt ein, so kann der Stecker 9 um die durch diesen Eingriff definierte Drehstelle nach unten geklappt werden, um die Steckerkanüle 10 in das Gehäuse 5 durch das Dichtelement 4 hindurch einzuführen und so einen Fluss einer Flüssigkeit von der Flüssigkeitsfördereinrichtung durch den Kupplungsteil 13, den Schlauch 12, den Stecker 9 und die Steckerkanüle 10 in die Kanüle 3 und somit in das umliegende Gewebe zu ermöglichen.

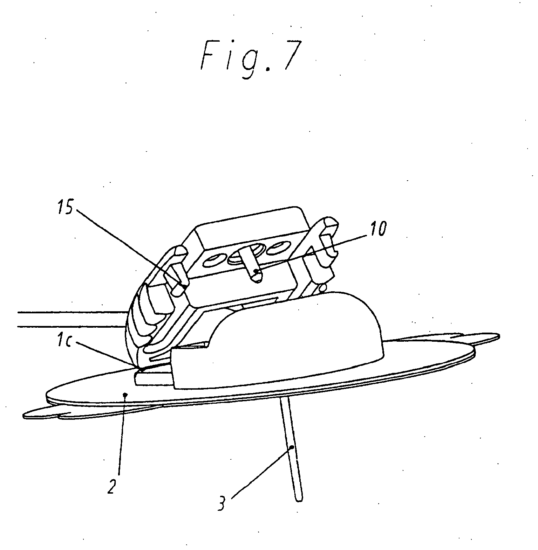

Wird der Stecker 9 aus der in den Figuren 6 und 7 gezeigten Position nach unten geklappt, so erfolgt eine Führung des Steckers 9 über Führungsnocken 14, um die Steckerkanüle 10 positionsgenau in das Gehäuse 5 einzuführen. Durch die Führung mittels der Führungsnocken 14 kann ein möglicherweise beim Ansetzen des Steckers 9 an die Kante 1b des Grundkörpers 1 vorliegender seitlicher Versatz des Steckers 9 relativ zum Grundkörper 1 beim Klappen des Steckers 9 korrigiert werden, so dass die Steckerkanüle 10 immer sicher in das Gehäuse 5 eingeführt werden kann.

Im vollständig heruntergeklappten Zustand des Steckers 9, in welchem die Steckerkanüle 10 in das Gehäuse 5 eingeführt ist, verrasten die seitlich am Stecker 9 vorgesehenen Rastvorrichtungen 15 mit den in Figur 2 gezeigten Rastnasen 1b des Grundkörpers 1, wodurch der Stecker 9 sicher mit dem Grundkörper 1 verbunden ist.

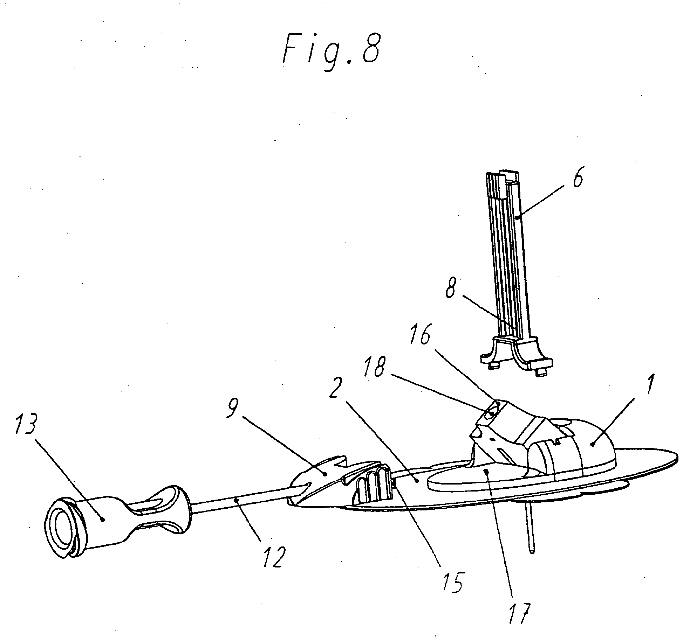

Figur 8 zeigt eine alternative Ausführungsform. Ein in dem Grundkörper 1 drehbar gelagertes Drehteil 16 kann in eine Position gebracht werden, bei welcher die Öffnung 18 des Drehteiles 16 nach oben weist. In dieser Position kann mit Hilfe der durch einen als Schutzelement 6 dienenden Rahmen geschützten Führungsnadel 8 eine Kanüle in den Grundkörper 1 eingebracht werden, wie oben beschrieben. Nach einbringen der Kanüle kann die Führungsnadel 8 wieder entfernt werden. Das Drehteil 16 kann nun, wie in Figur 8 gezeigt, auf die Seite gedreht werden und verrastet bevorzugt mit einer auf der Grundebene des Grundkörpers 1 angebrachten nach oben weisenden Rastnase 17, wodurch das Drehteil 16 sicher in seiner heruntergeklappten Position gehalten wird. Ein Stecker 9 kann bei dieser alternativen Ausführungsform seitlich an dem vollständig heruntergeklappten Drehteil 16 so angebracht werden, dass die in gerader Verlängerung des Schlauches 12 verlaufende Steckerkanüle 10 in die auf die Seite weisende Öffnung 18 des Drehteiles 16 eingebracht werden kann. Dabei kann eine an dem Stecker 9 angebrachte Rastvorrichtung 15 mit geeigneten Gegenstücken des Drehteiles 16 verrasten.

Bei den oben beschriebenen Ausführungsformen kann der Stecker 9 jeweils wieder durch seitlichen Druck auf die oberhalb oder seitlich der Rastvorrichtungen 15 des Steckers 9 liegenden Bereiche wieder vom Grundkörper 1 diskonnektiert werden. Nach erfolgter Diskonnektion schließt das Dichtelement 4 den Zugang zur Kanüle 3 wieder vollständig.

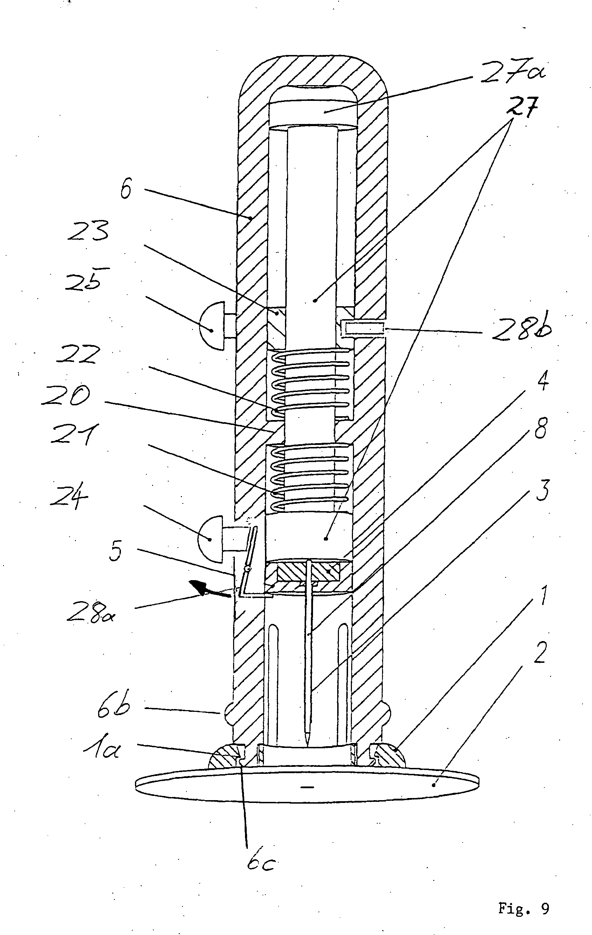

Figur 9 zeigt eine automatische Kanüleneinbringvorrichtung. Bezüglich der Beschreibung des Grundkörpers 1 mit zugehörigen Rastverbindungen und dem Einbringen der Kanüle 3 mit Halterung 5 und Dichtelement 4 wird auf die voranstehende Beschreibung verwiesen. In dem Führungselement 6 ist ein in Längsrichtung des Führungselements 6 bewegbarer Nadelträger 27 vorgesehen, welcher fest mit der Führungsnadel 8 verbunden ist. Alternativ kann die Führungsnadel 8 auch mit dem Nadelträger 27 koppelbar ausgestaltet sein, um z. B. die Führungsnadel 8 wechseln zu können. Der Nadelträger 27 befindet sich in einem zurückgezogenen Zustand und wird durch eine an der Vorderseite des Nadelträgers 27 anliegende Einbringfeder 21 vorgespannt, welche sich gegen ein etwa im Mittelteil des Führungselements 6 vorgesehenes Stützelement 20 abstützt und den durch das Halteelement 28a gesicherten Nadelträger 27 in Ausschubrichtung der Kanüle 3 vorspannt. Das Halteelement 28a kann durch einen ersten Auslöseknopf 24 entsichert werden. Auf der entgegengesetzten Seite des Stützelements 20 ist eine Rückzugfeder 22 vorgesehen, welche gegen einen durch ein Halteelement 28b gesicherten Haltering 23 drückt, der durch einen zweiten Auslöseknopf 25 entsichert werden kann. Der Nadelträger 27 verläuft in Längsrichtung des Führungselements 6 durch die beiden Federn 21 und 22, sowie das Stützelement 20 und den Haltering 23 verschieblich hindurch und weist an seinem hinteren Ende ein Halteelement 27a auf, um zu verhindern, dass der Nadelträger 27 durch den Haltering 23 vollständig hindurchtreten kann. Wird der Auslöseknopf 24 gedrückt, so wird das schematisch gezeichnete Halteelement 28a in Pfeilrichtung radial nach außen geschoben bzw. geklappt, wobei der Klappmechanismus bevorzugt vollständig innerhalb des Führungselements 6 ausgebildet ist, wobei dann der Nadelträger 27 in Ausschubrichtung der Kanüle 3 nicht mehr gehalten wird und durch die Kraft der Einbringfeder 21 nach unten beschleunigt wird, um mit der Führungsnadel 8 die Kanüle 3 aus dem Führungselement 6 auszustoßen und durch das Pflaster 2 hindurch in ein Gewebe einzubringen. Dabei wird die Ausstoßbewegung fortgesetzt, bis das Halteelement 27a des Nadelträgers 27 an dem Haltering 23 anliegt.

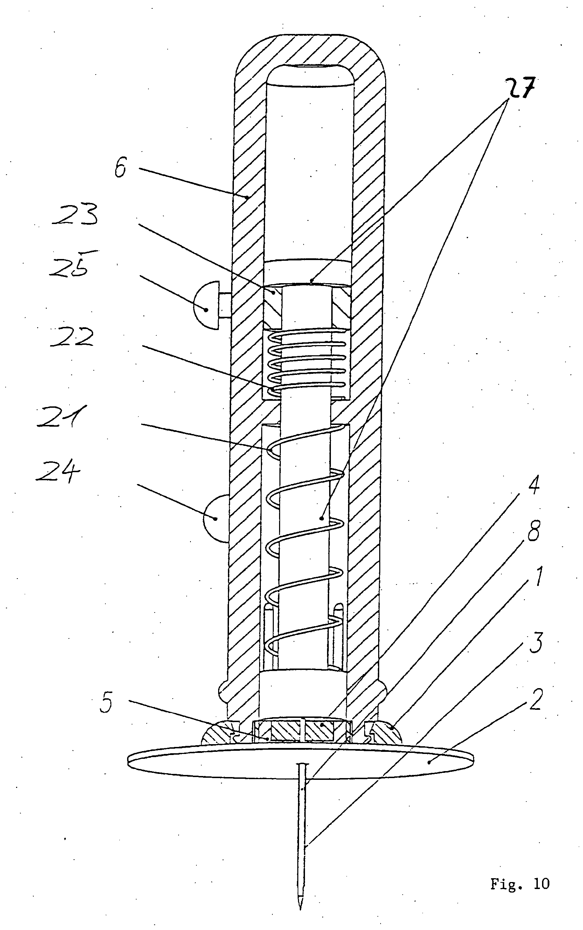

Figur 10 zeigt die in Figur 9 gezeigte Kanüleneinbringvorrichtung nach erfolgtem Ausschubvorgang der Kanüle 3. Die Einbringfeder 21 hat die in ihr gespeicherte Energie auf den Nadelträger 27 übertragen und ist im entspannten Zustand. Die Halterung 5 kann z. B. wie oben beschrieben, mit dem Grundkörper 1 verrastet werden. Wird vom Benutzer nun der zweite Auslöseknopf 25 betätigt, so wird das radial verschiebbare Halteelement 28b (Fig. 9) aus dem Haltering 23 herausgeschoben und der Haltering 23, welcher bisher gegen axiale Bewegung im Führungselement 6 gesichert war, freigegeben, so dass die Rückzugfeder 22 gegen den Haltering 23 und das Halteelement 27a des Nadelträgers 27 drückt und somit eine Rückzugkraft auf den Nadelträger 27 wirkt. Bevorzugt ist die Rückzugfeder 22 in der gezeigten Ausführungsform so ausgestaltet, dass diese eine stärkere Kraft aufbringen kann, als die Einbringfeder 21, da diese beim Zurückziehen des Nadelträgers 27 wieder zusammengedrückt wird. Alternativ kann der Nadelträger 27 auch so ausgebildet sein, dass nach Einbringen der Kanüle 3 keine Kopplung zwischen unterer Seite des Nadelträgers 27 und Einbringfeder 21 mehr vorliegt, so dass beim Zurückziehen des Nadelträgers 27 die Einbringfeder 21 nicht mehr gespannt werden muss.

Allgemein kann das Halteelement 28a und/oder das Halteelement 28b als Kipp- oder Schiebemechanismus oder als ein beliebiger anderer Sicherungsmechanismus ausgebildet sein.

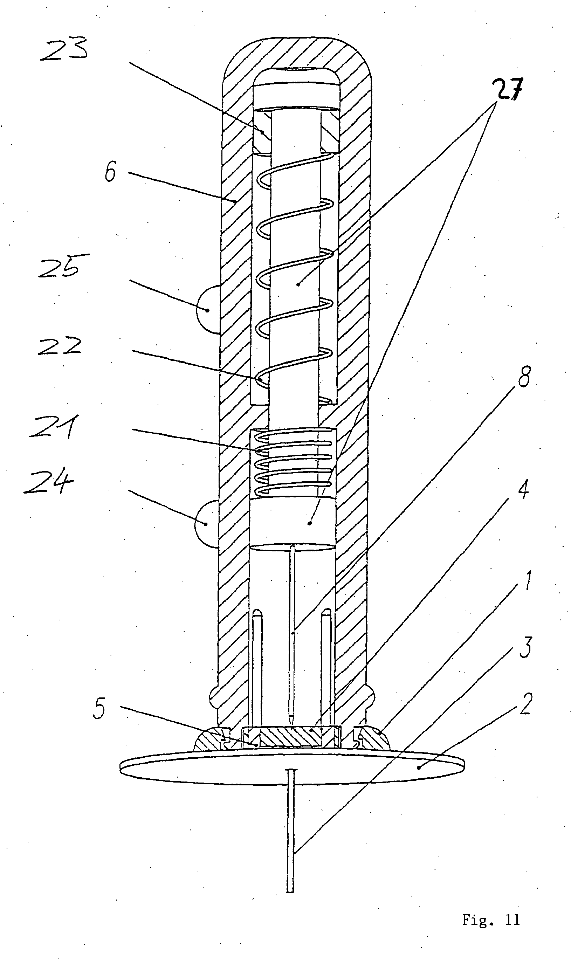

Figur 11 zeigt die in Figur 10 gezeigte Kanüleneinbringvorrichtung nach erfolgtem Rückziehen des Nadelträgers 27. Die Führungsnadel 8 wurde dabei aus der ausgeschobenen Kanüle 3 wieder zurückgezogen und in das Führungselement 6 eingebracht, um Verletzungen zu vermeiden.

Alternativ zu der gezeigten Ausführungsform kann z. B. das Zurückziehen des Nadelträgers 27 voll automatisch nach Einbringen der Kanüle 3 erfolgen, indem z. B. durch die Unterseite des Nadelträgers 27 ein Auslösemechanismus für die Rückzugfeder 22 betätigt wird, wodurch unmittelbar nach Einbringen der Kanüle 3 der Nadelträger 27 wieder zurückgezogen wird. In diesem Fall kann dann der zweite Auslöseknopf 25 entfallen.

Gemäß einer weiteren alternativen Ausführungsform kann die Auslösung der Rückzugfeder 22 durch Entsichern des Halterings 23 automatisch erfolgen, wenn die Kanüleneinbringvorrichtung von dem Grundkörper 1 abgenommen wird, z. B. indem durch das Eindrücken der Knöpfe 6b um die Verrastung der Rastnasen 6c des Führungselements 6 mit den Rastnasen 1a des Grundkörpers 1 zu lösen auch gleichzeitig der Haltering 23 entsichert wird.

Vorteilhaft ist die Kanüleneinbringvorrichtung so ausgestaltet, dass das Lösen der Verrastung zwischen Führungselement 6 und Grundkörper 1 nicht erfolgen kann, wenn die Führungsnadel 8 in der in Figur 9 gezeigten Positionen vor dem Ausschieben und/oder in der in Figur 10 gezeigten ausgeschobenen Position ist. Dies kann z. B. dadurch erfolgen, dass die Unterseite des Nadelträgers 27 so breit ist, dass ein Zusammendrücken der Unterseite des Führungselements 6 z. B. an den Knöpfen 6b verhindert wird und in der in Figur 10 gezeigten Stellung somit die Verrastung zwischen Führungselement 6 und Grundkörper 1 nicht gelöst werden kann.

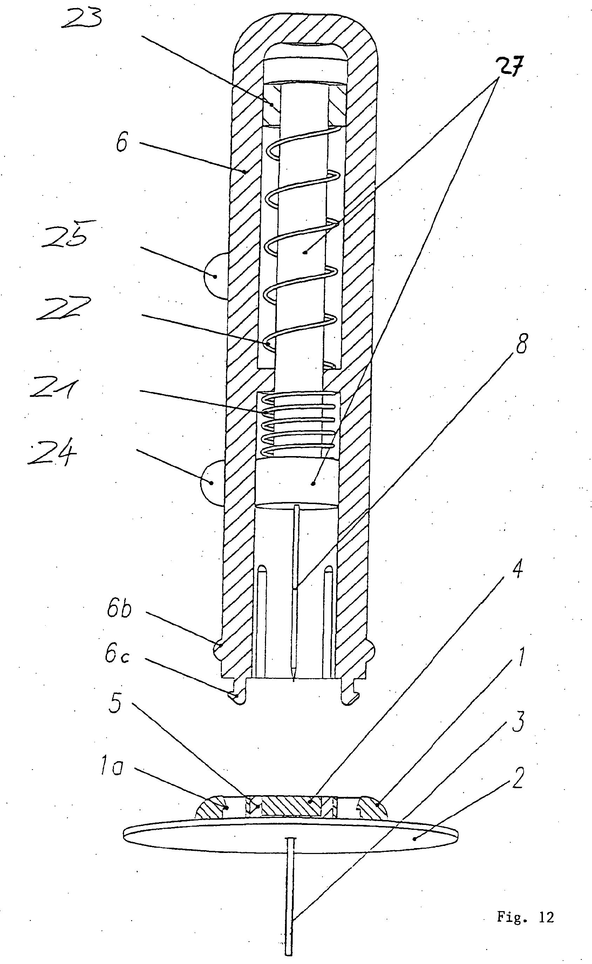

Figur 12 zeigt die von dem Grundkörper 1 abgelöste Kanüleneinbringvorrichtung mit zurückgezogener Führungsnadel 8. Die Kanüleneinbringvorrichtung kann nun gefahrlos entsorgt werden, da die Führungsnadel 8 durch das umliegende Führungselement 6 abgedeckt ist und somit die Gefahr unbeabsichtigter Stichverletzungen minimiert ist. Alternativ kann durch nicht gezeigte Spannvorrichtungen die Kanüleneinbringvorrichtung wieder in den in Figur 9 gezeigten Zustand gebracht werden, indem z. B. der Haltering 23 aus der oberen Position wieder in eine untere Position geschoben wird, wobei die Rückzugfeder 22 wieder gespannt wird. Ebenso ist es möglich den Nadelträger 27 aus dem Führungselement 6 auszuschieben und dabei die Rückzugfeder 22 zu spannen, wobei anschließend in einem zweiten Schritt die Einbringfeder 21 wieder gespannt wird. Bevorzugt wird bei einer Mehrwegvorrichtung die benutzte Führungsnadel ausgewechselt und durch eine neue Führungsnadel, gegebenenfalls mit neuer Kanüle 3 und zugehöriger Halterung 5 mit Dichtelement 4 ersetzt.

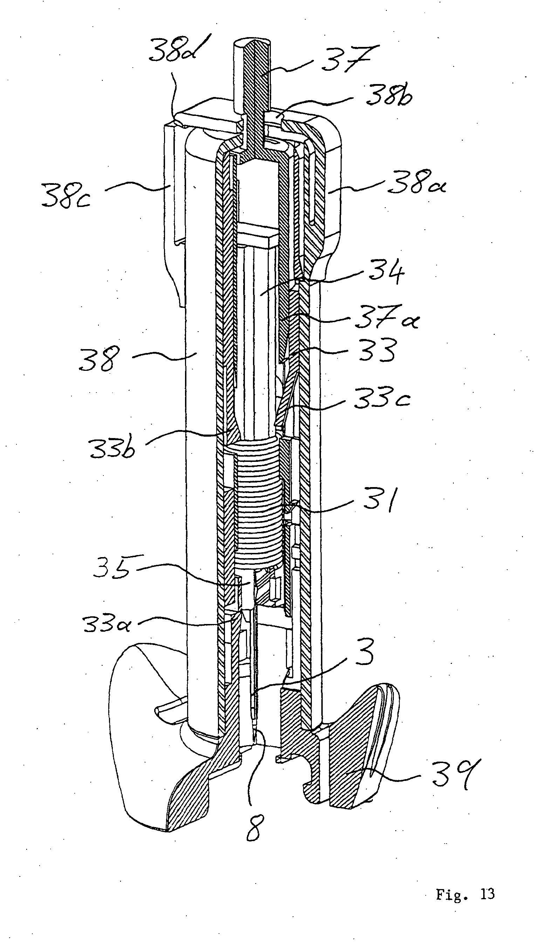

Figur 13 zeigt eine erste Ausführungsform einer automatischen Kanüleneinbring- und Rückzugsvorrichtung im Ausgangszustand vor dem Einbringen einer Kanüle 3 an einer Kanülenbaugruppe 35 durch eine Führungsnadel 8. Die Kanüleneinbringvorrichtung weist eine Konnektorhülse bzw. ein Führungselement 38 auf, welches an seinem unteren Ende Konnektorelemente 39 hat, um beispielsweise auf einem Grund- oder Basiskörper 1 befestigt zu werden, wie für eine der Ausführungsformen in den vorangehenden Figuren gezeigt. Diese Konnektorelemente 39 zum Verbinden der Konnektorhülse 38 mit einem vorgegebenen Basiskörper weisen beispielsweise Rastzungen 39a und weitere nicht gezeigte Verbindungselemente auf. Innerhalb der Konnektorhülse 38 ist die Führungshülse 33 mit davon nach innen vorstehenden Zungen 33a, 33b und 33c vorgesehen. Die Zungen 33a bis 33c sind an verschiedenen Positionen in axialer Richtung der Führungshülse 33 angeordnet und können als einzelne vorstehende Elemente oder auch über einen größeren Teil des Umfangs der Führungshülse 33 angeordnet sein. Die Zungen können beispielsweise auch einander gegenüberliegen und/oder symmetrisch zueinander als eine Mehrzahl von einzelnen Zungenelementen ausgebildet sein. Die drei beispielhaft gezeigten Zungen 33a bis 33c sind in axialer Richtung der Führungshülse 33 versetzt zueinander angeordnet, um verschiedene Funktionen beim Auslösen eines Ausschub- und Rückzug-Vorgangs zu erfüllen, wie nachfolgend erläutert werden wird. Innerhalb der Führungshülse 33 ist fest mit dem Auslöseknopf 37 verbunden eine Auslösehülse 37a vorgesehen, welche in axialer Richtung Ausnehmungen aufweist, die den Zungen 33b und 33c zugeordnet sind. Weiterhin sind an der Auslösehülse 37a spezielle Auslöseoberflächen, wie beispielsweise Abschrägungen vorgesehen, um die Zungen 33a bis 33c bei einem Verschieben der Auslösehülse 37a nach unten in der in Figur 13 gezeigten Ausführungsform in einer vorgegebenen Reihenfolge wegzudrücken, wodurch der Ausschub- und Rückzug-Vorgang der Kanülenbaugruppe 35 eingeleitet wird. Innerhalb der Konnektorhülse 38 ist weiterhin ein Nadelteil oder Nadelträger 34 verschiebbar angeordnet, welcher über eine fest verbundene Führungsnadel 8 und bevorzugt eine direkte Anlagefläche mit der Kanülenbaugruppe 35 gekoppelt oder über eine Rastvorrichtung (nicht gezeigt) mit dieser verrastet ist. Die vollständig vorgespannt Feder 31 drückt im gezeigten Ausgangszustand auf die Kanülenbaugruppe 35, wobei ein Paar von oberhalb der Feder 31 symmetrisch angeordneten Zungen 33b und ein Paar von unterhalb der Kanülenbaugruppe 35 angeordneten Zungen 33a der Führungshülse 33 die auf die Kanülenbaugruppe 35 drückende vorgespannte Feder 31 in Position halten. An der Oberseite der Konnektorhülse 38 ist ein Sicherungsbügel 38a mit einer darin vorgesehenen Bohrung 38b vorgesehen, wobei der Durchmesser der Bohrung 38b etwa dem Durchmesser des Auslöseknopfes 37 entspricht und bevorzugt ein wenig größer ist, um ein leichtes Hindurchtreten des Auslöseknopfes 37 zu ermöglichen. Der in etwa L-förmig ausgebildete Sicherungsbügel 38a liegt einem Sicherungsbügel 38c mit einem elastischen Element an der Oberseite der Konnektorhülse 38 gegenüber, wobei eine an dem Sicherungsbügel 38a vorgesehene Zunge 38d mit dem Sicherungsbügel 38c verrasten kann, wenn der Sicherungsbügel 38a und das Sicherungsbügel 38c zusammengedrückt werden. Die in Figur 13 gezeigte zur Kanülenachse exzentrisch angeordnete Bohrung 38b verhindert, dass der Auslöseknopf 37 gedrückt werden kann. Werden der Sicherungsbügel 38a und das Sicherungsbügel 38c so zusammengedrückt, dass der Sicherungsbügel 38c z. B. mit der Zunge 38d einrastet, so wird die Bohrung 38b in eine Position konzentrisch zur Position des Auslöseknopfes 37 gebracht, so dass dieser gedrückt werden kann.

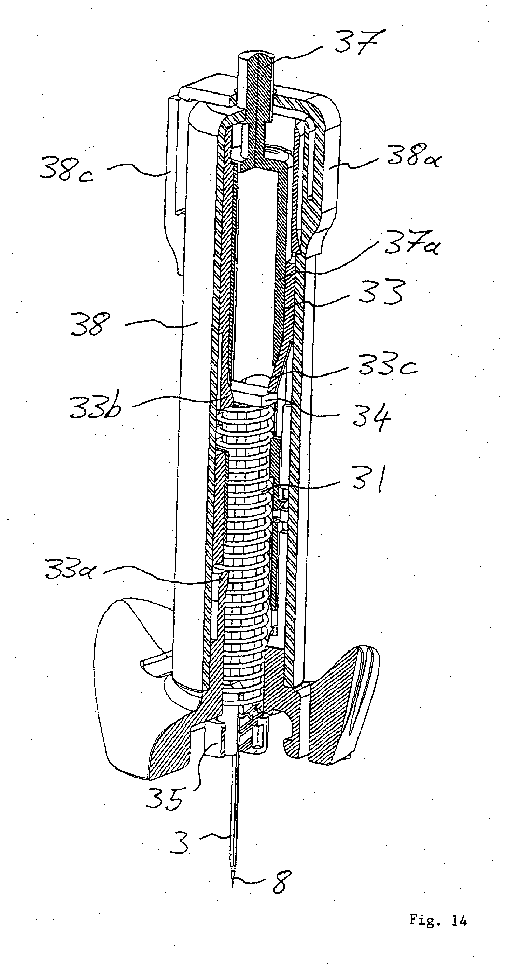

Figur 14 zeigt den Zustand der in Figur 13 gezeigten Vorrichtung, nachdem der Betätigungsknopf 37 um etwa eine halbe Länge eingedrückt wurde. Die unteren Zungen 33a, auf welche die Kanülenbaugruppe 35 durch die Feder 31 gedrückt wurde, werden durch die sich entlang der Innenseite der Konnektorhülse 38 erstreckende Auslösehülse 37a deformiert, wodurch die Kanülenbaugruppe 35 zusammen mit dem Nadelträger 34 freigegeben wird und von der Feder 31 an einen unteren Endanschlag, welcher in der Kanüleneinbringvorrichtung oder an einem Basiskörper 1 vorgesehen sein kann, gedrückt wird. Das obere Ende des Nadelträgers 34 führt dabei über die Zungen 33c. Die mit der Führungsnadel 8 verbundene Kanüle 3 der Kanülenbaugruppe 35 wird durch die Kraft der Feder 31 nach unten bevorzugt mit hoher Geschwindigkeit aus der Vorrichtung ausgeschoben und kann wie gewünscht in ein Gewebe eingebracht werden.

In der in Figur 14 gezeigten Position ist die Kanülenbaugruppe 35 mit einem (nicht gezeigten) Grundkörper oder Basiskörper eines Infusionssets verriegelt und der Nadelträger 34 wird durch Zungen im Grundkörper des Infusionssets entriegelt, so dass Nadelträger 34 und Kanülenbaugruppe nicht mehr verbunden sind (in Figur 14 nicht gezeigt). Dabei ist der Nadelträger 34 in dieser Position durch die zwei symmetrisch zueinander angeordneten Zungen 33c verriegelt, was ein Umplazieren des Sets möglich macht.

Die Feder 31 im halbentspannten Zustand drückt unten gegen die ausgeschobene Kanülenbaugruppe 35 und liegt an der gegenüberliegenden Seite an einem vorstehenden Element des Nadelteiles 34 an, wobei das Nadelteil 34 gegen eine axiale Verschiebung durch die Zungen 33c gesichert ist. Die Feder 31 wird in der gezeigten Position durch die Zungen 33b gehalten. Allgemein kann die Feder auch in der gezeigten Position z.B. von dem oberen Ende des Nadelteiles 34 gehalten werden.

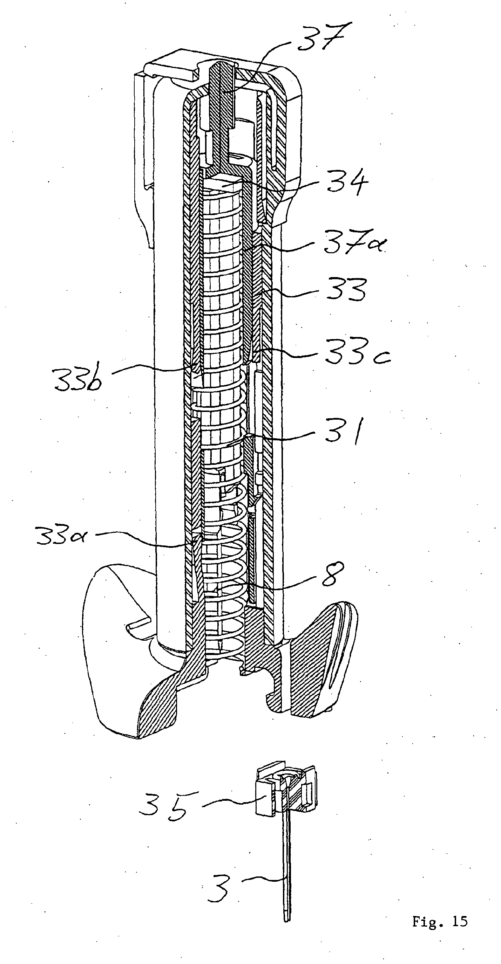

Figur 15 zeigt den Zustand der Vorrichtung von Figur 14 , nachdem der Betätigungsknopf 37 ganz eingedrückt wurde. Hierdurch wird die Auslösehülse 37a über die Zungen 33b und 33c geschoben, welche nach außen weggedrückt werden und so das Nadelteil 34 freigeben, welches durch die Feder 31, welche sich nun voll entspannen kann, wieder zurück in die Konnektorhülse 38 geschoben wird. Hierdurch wird die mit dem Nadelteil 34 verbundene Führungsnadel 8 aus der Kanüle 3 und der Kanülenbaugruppe 35 in die Konnektorhülse 38 zurückgezogen, wobei die Kanüle 3 z. B. in einem Gewebe verbleiben kann. Das Nadelteil 34 wird durch die Feder 31 in der zurückgezogenen Position erhalten, so dass die Führungsnadel 8 nicht unbeabsichtigt aus der Konnektorhülse 38 austreten kann, wodurch das Verletzungsrisiko minimiert wird. Die Konnektorhülse 38 kann nun von einem Basiskörper durch Lösen der Konnektorelemente 39 abgenommen werden.

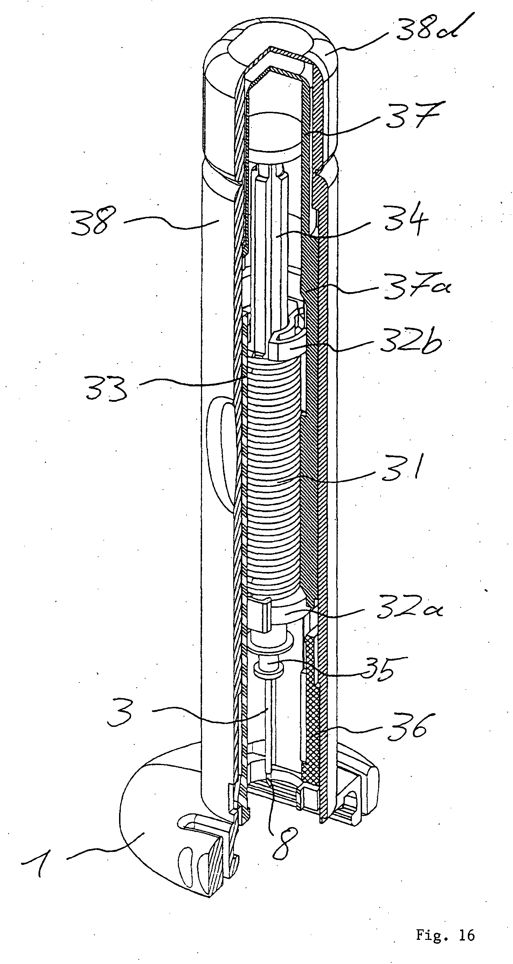

Figur 16 zeigt eine zweite Ausführungsform einer automatischen Kanüleneinbring- und Rückzugsvorrichtung im Ausgangszustand mit einer Konnektorhülse 38, welche mit einem Grundkörper 1 verbunden ist. Ein Auslöseknopf 37 geht in die Auslösehülse 37a über, welche innerhalb der Konnektorhülse 38 angeordnet ist. Die Auslösehülse 37a weist entlang ihrer axialen Richtung an der Innenseite Vertiefungen und untere und obere Abschrägungen auf, mit welchen bei axialer Verschiebung der Auslösehülse 37a innerhalb der Konnektorhülse 38 ein unterer und ein oberer Mitnehmerring 32a und 32b entkoppelt werden können, um die Führungsnadel 8 aus der Konnektorhülse 38 zusammen mit der um diese angeordneten Kanüle 3 mit zugehöriger Kanülenbaugruppe 35 auszuschieben und anschließend die Führungsnadel 8 aus der Kanüle 3 und der Kanülenbaugruppe 35 zurückzuziehen, wie nachfolgend beschrieben werden wird.

Der untere Mitnehmerring 32a liegt an einer Anschlaghülse 36 an und wird von dieser gegen den Druck der Feder 31 in Position gehalten. Die Feder 31 drückt an der Oberseite gegen den oberen Mitnehmerring 32b, welche durch die Führungshülse 33 z. B. in einer Rille oder Vertiefung gehalten wird. Der Nadelträger 34 ist so ausgestaltet, dass er in axialer Richtung der Konnektorhülse 38 durch die Mitnehmerringe 32a und 32b hindurch bewegt werden kann bis zum Ende des Nadelträgers 34, welcher einen größeren Durchmesser aufweist als der Innendurchmesser der Mitnehmerringe, um so zu verhindern, dass der Nadelträger 34 beispielsweise herausfallen kann. Der Nadelträger 34 ist wiederum fest mit der Führungsnadel 8 verbunden.

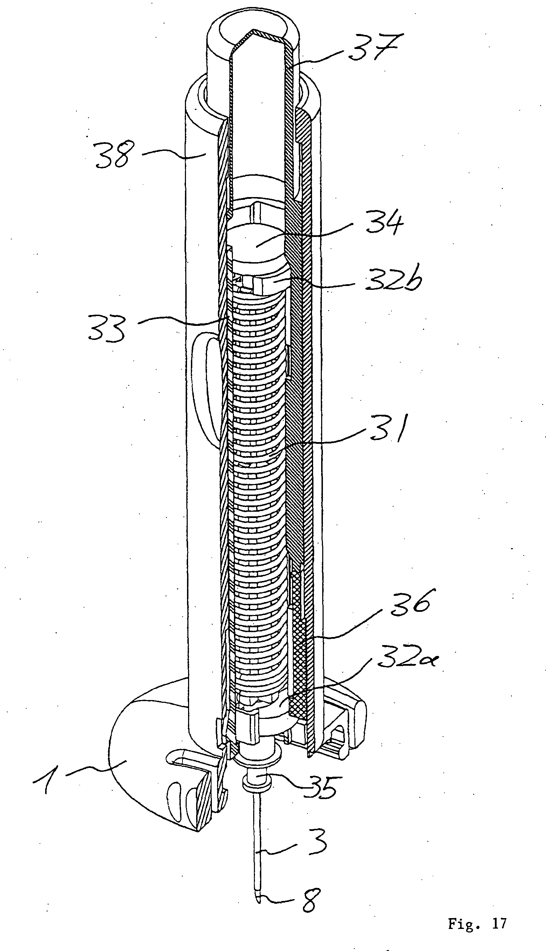

Figur 17 zeigt die in Figur 16 gezeigte Vorrichtung, nachdem die über dem Auslöseknopf 37 angeordnete Sicherungskappe 38d abgenommen und der Auslöseknopf 37 um etwa eine halbe Länge eingedrückt worden ist. Der untere Mitnehmerring 32a wird durch eine untere schräge innere Oberfläche der Auslösehülse 37a seitlich weg von der Anschlaghülse 36 verschoben und damit von dieser entkoppelt. Dabei kann der untere Mitnehmerring 32a entweder schon mit dem Nadelträger 34 gekoppelt sein oder durch diesen Verschiebevorgang mit dem Nadelträger 34 gekoppelt werden. Die Feder 31 drückt nun gegen den noch fest durch die Führungshülse 33 gehaltenen oberen Mitnehmerring 32b und drückt den mit dem Nadelträger 34 gekoppelten unteren Mitnehmerring 32a nach unten und bringt so die Führungsnadel 8 zusammen mit der Kanüle 3 in ein unterhalb des Grundkörpers 1 liegendes Gewebe ein, wobei wiederum die Kanülenbaugruppe 35 bis zu einem unteren Endanschlag bewegt wird. In dieser Position bleibt der Nadelträger 34 mit dem Grundkörper 1 verriegelt, was ein Umplatzieren des gesamten Sets möglich macht.

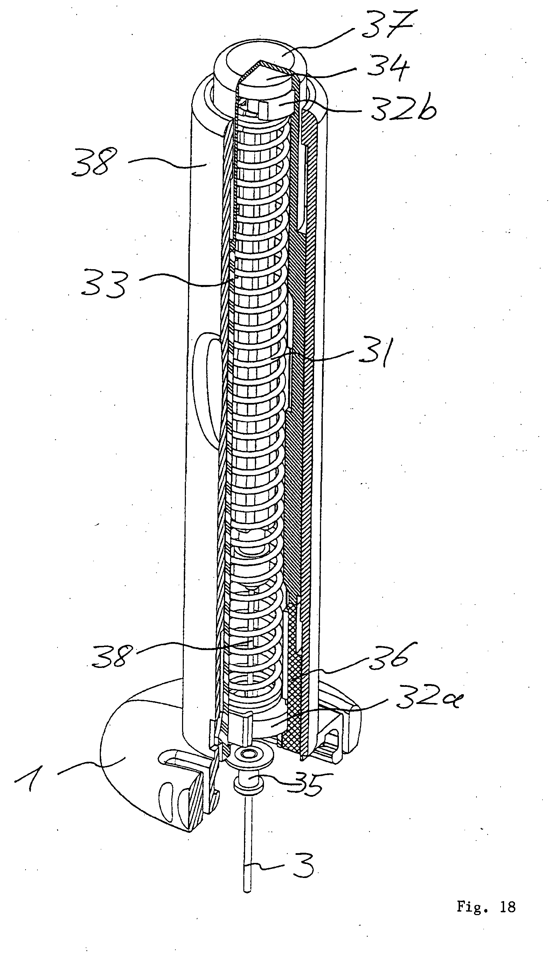

Figur 18 zeigt den Zustand der in Figur 17 gezeigten Vorrichtung, nachdem der Auslöseknopf 37 weiter eingedrückt wurde. Dabei wird zuerst der Nadelträger 34 vollständig von der Kanülenbaugruppe 35 entriegelt. Der obere Mitnehmerring 32b wird durch eine obere schräge Fläche der Auslösehülse 37a seitlich verschoben und so von der Führungshülse 33 entkoppelt und koppelt in den Nadelträger 34, so dass die Feder 31 über den oberen Mitnehmerring 32b den Nadelträger 34 nach oben bis zu einem oberen Endanschlag drücken kann. Hierdurch wird die Führungsnadel 8 aus der Kanüle 3 und der Kanülenbaugruppe 35 zurückgezogen und wird durch die gegen den Nadelträger 34 drückende Feder 31 in der zurückgezogenen Position gehalten, wodurch das Verletzungsrisiko minimiert wird. Allgemein können zum Auslösen der Ausschub- und Rückzieh-Vorgänge ein oder mehrere Betätigungselemente, z. B. Knöpfe vorgesehen sein, welche direkt oder indirekt, z. B. über eine Verschiebung eines Elements in der Kanüleneinbringvorrichtung, den entsprechenden Vorgang z. B. über das Freigeben einer Feder, auslösen.

## Patentansprüche

1. Ein System zum Verbinden einer Flüssigkeitszufuhr mit einer Kanüle, umfassend:
einen Basiskörper, bestehend aus einem Grundkörper (1) und einem Pflaster (2), wobei der Grundkörper auf einer ersten Oberfläche des Pflasters aufgebracht ist, und wobei der Grundkörper mindestens eine Öffnung aufweist, und das Pflaster eine zweite, entgegengesetzte, klebende Oberfläche aufweist zum Aufkleben des Basiskörpers auf einer Hautstelle; und
eine separate Halterung (5, 35), die fest mit einer Kanüle (3) zum Verabreichen einer Flüssigkeit in ein Gewebe verbunden ist;
wobei die Halterung und der Grundkörper dazu konfiguriert sind, bei der Einführung der Kanüle durch den Grundkörper hindurch in ein Gewebe eine Verbindung zu bilden;
und einen Stecker (9) mit einem Zuführelement (10) als Vorrichtung zum Zuführen einer Flüssigkeit.

2. System nach Anspruch 1, **dadurch gekennzeichnet, dass** die Vorrichtung zum Zuführen einer Flüssigkeit über Verbindungselemente mit dem Grundkörper verbindbar ist, wobei die Verbindung bevorzugt lösbar ausgebildet ist.

3. System nach Anspruch 2, **dadurch gekennzeichnet, dass** die Verbindungselemente sowohl zur Verbindung mit einer Einbringvorrichtung für die Kanüle, als auch zur Verbindung mit der Vorrichtung zum Zuführen einer Flüssigkeit ausgebildet sind.

4. System nach einem der vorherigen Ansprüche, **dadurch gekennzeichnet, dass** das Zuführelement in die Öffnung des Grundkörpers einbringbar ist, so dass die Flüssigkeit über das Zuführelement durch die Öffnung des Grundkörpers in den Kanülenhohlraum leitbar ist.

5. System nach Anspruch 4, **dadurch gekennzeichnet, dass** der Stecker an einem Anlagepunkt, einer Anlagekante mit geradem oder gebogenem oder abgewinkeltem Verlauf oder einer Anlagefläche des Grundkörpers anlegbar und um diese klappbar gestaltet ist.

6. System nach einem der vorherigen Ansprüche, **dadurch gekennzeichnet, dass** ein Führungselement am Grundkörper und/oder am Stecker vorgesehen ist.

7. System nach einem der vorherigen Ansprüche, **dadurch gekennzeichnet, dass** der Stecker mit dem Grundkörper mittels Rastverbindung verbindbar ist, wobei vorzugsweise geeignete Nuten oder Rastnasen vorgesehen sind.

8. System nach Anspruch 7, **dadurch gekennzeichnet, dass** die Rastverbindung lösbar oder unlösbar ausgestaltet ist.

9. System nach einem der Ansprüche 1 bis 3, **dadurch gekennzeichnet, dass** der Grundkörper ein drehbar gelagertes Drehteil aufweist, welches bevorzugt fest mit dem Grundkörper verbunden ist.

10. System nach Anspruch 9, **dadurch gekennzeichnet, dass** das Drehteil eine bevorzugt durch ein Dichtungselement abgeschlossene Öffnung aufweist, in welche in einer ersten Position des Drehteils eine Kanüle einführbar und in einer zweiten gedrehten Position des Drehteils ein Zuführelement einer Vorrichtung zum Zuführen einer Flüssigkeit anschliessbar ist.

11. System nach Anspruch 10, **dadurch gekennzeichnet, dass** an das Drehteil in der zweiten gedrehten Position seitlich, bei nicht nach oben weisender Öffnung des Drehteils das Zuführelement anschliessbar ist.

## Claims

1. System for connecting a fluid supply with a cannula, comprising:
a base body consisting of a main body (1) and a sticking plaster (2), wherein the main body is placed on a first surface of the sticking plaster, and wherein the main body has at least one opening, and the sticking plaster has a second, adhesive surface on the opposite side for sticking the base body to an area of skin; and
a separate mounting (5, 35) which is fixedly connected to a cannula (3) for introducing a fluid into a tissue; wherein the mounting and the main body are configured so as to create a connection when the cannula is introduced into a tissue through the main body;
and a plug (9) with a feed element (10) as an apparatus for supplying a fluid.

2. System according to Claim 1, **characterized in that** the apparatus for supplying a fluid can be connected to the main body via connector elements, wherein the connection is designed to be detachable.

3. System according to Claim 2, **characterized in that** the connector elements are designed to form a connection both with an insertion apparatus for the cannula and a connection with the apparatus for delivering a fluid.

4. System according to any one of the preceding claims, **characterized in that** the feed element can be introduced into the opening in the main body so that the fluid can be transported through the opening in the main body via the feed element, and into the cannula cavity.

5. System according to Claim 4, **characterized in that** the plug can be placed on a contact point, a contact edge with straight or curved or angled alignment or on a contact surface of the main body and is designed such that it can be folded around them.

6. System according to any one of the preceding claims, **characterized in that** a guide element is provided on the main body and/or on the plug.

7. System according to any one of the preceding claims, **characterized in that** the plug can be connected to the main body by means of detent connection, wherein preferably suitable grooves or detent lugs are provided.

8. System according to Claim 7, **characterized in that** the detent connection is designed to be detachable or non-detachable.

9. System according to any one of Claims 1 to 3, **characterized in that** the main body has a rotatably mounted rotary member, which is preferably fixedly connected to the main body.

10. System according to Claim 9, **characterized in that** the rotary member has an opening which is preferably closed by a sealing element, and into which a cannula can be inserted in a first position of the rotary member, and in a second, rotated position of the rotary member a feed element of an apparatus device can be attached for supplying a fluid.

11. System according to Claim 10, **characterized in that** the feed element can be connected laterally to the rotary member in the second, rotated position when the opening is not facing upwards.

## Revendications

1. Un système, destiné à relier une alimentation de liquide sur une canule, comprenant : un corps de base, constitué d'un corps principal (1) et d'un pansement (2), le corps principal étant appliqué sur une première surface du pansement et le corps principal comportant au moins un orifice et le pansement comportant une deuxième surface opposée adhésive, prévue pour coller le corps de base sur une zone cutanée ; et
une fixation (5, 35) séparée, qui est fixement reliée avec une canule (3), pour l'administration d'un liquide dans un tissu ;
la fixation et le corps principal étant configurés pour former une liaison, lors de l'introduction de la canule à travers le corps principal dans un tissu ;
et un connecteur (9) pourvu d'un élément d'alimentation (10) faisant office de dispositif d'alimentation d'un liquide.

2. Système selon la revendication 1, **caractérisé en ce que** le dispositif d'alimentation d'un liquide est susceptible d'être relié avec le corps principal par l'intermédiaire d'éléments de liaison, la liaison étant conçue de préférence de manière amovible.

3. Système selon la revendication 2, **caractérisé en ce que** les éléments de liaison sont conçus aussi bien pour la liaison avec un dispositif d'introduction pour la canule, qu'également pour la liaison avec le dispositif destiné à alimenter un liquide.

4. Système selon l'une quelconque des revendications précédentes, **caractérisé en ce que** l'élément d'alimentation est susceptible d'être introduit dans l'orifice du corps principal, de telle sorte que le liquide puisse être dirigé par l'intermédiaire de l'élément d'alimentation à travers l'orifice du corps principal dans la cavité de la canule.

5. Système selon la revendication 4, **caractérisé en ce que** le connecteur est conçu pour pouvoir s'appuyer sur un point d'appui, sur une arête d'appui ayant un trajet rectiligne ou curviligne ou coudé ou sur une surface d'appui du corps principal et pour basculer autour de ceux-ci.

6. Système selon l'une quelconque des revendications précédentes, **caractérisé en ce qu'**un élément de guidage est prévu sur le corps principal et/ou sur le connecteur.

7. Système selon l'une quelconque des revendications précédentes, **caractérisé en ce que** le connecteur est susceptible d'être relié avec le corps principal au moyen d'une liaison par enclenchement, de préférence, des rainures ou des becs d'enclenchement adaptés étant prévus.

8. Système selon la revendication 7, **caractérisé en ce que** la liaison par enclenchement est prévue pour être amovible ou inamovible.

9. Système selon l'une quelconque des revendications 1 à 3, **caractérisé en ce que** le corps principal comporte une pièce rotative logée en rotation, laquelle est reliée de préférence de manière fixe avec le corps principal.

10. Système selon la revendication 9, **caractérisé en ce que** la pièce rotative comporte un orifice fermé de préférence par un élément d'étanchéité, dans lequel dans une première position de la pièce rotative peut s'introduire une canule et dans une deuxième position tournée de la pièce rotative peut se raccorder un élément d'alimentation d'un dispositif destiné à alimenter un liquide.

11. Système selon la revendication 10, **caractérisé en ce que** dans la deuxième position tournée, lorsque l'orifice de la pièce rotative n'est pas dirigé vers le haut, l'élément d'alimentation peut se raccorder latéralement sur la pièce rotative.
